(19) Europäisches Patentamt
European Patent Office
Office européen des brevets

(11) EP 3 169 367 B1

(12) **EUROPEAN PATENT SPECIFICATION**

(45) Date of publication and mention of the grant of the patent:
**05.06.2019 Bulletin 2019/23**

(21) Application number: **15741260.2**

(22) Date of filing: **14.07.2015**

(51) Int Cl.:
***A61K 47/59*** *(2017.01)*

(86) International application number:
**PCT/GB2015/052028**

(87) International publication number:
**WO 2016/009190 (21.01.2016 Gazette 2016/03)**

(54) **POLY(BETA-AMINO ESTER) CONJUGATES FOR CARTILAGE AGENT DELIVERY AND METHODS**

POLY(BETA-AMINOESTER)-KONJUGATE ZUR KNORPELWIRKSTOFFABGABE UND VERFAHREN

CONJUGUÉS DE POLY(ESTER BÊTA-AMINO) POUR ADMINISTRATION D’UN AGENT CARTILAGINEUX ET PROCÉDÉS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **16.07.2014 GB 201412610**

(43) Date of publication of application:
**24.05.2017 Bulletin 2017/21**

(73) Proprietor: **University College Cardiff Consultants Ltd**
**Cardiff**
**South Glamorgan**
**CF24 0DE (GB)**

(72) Inventors:
- **PROKOPOVICH, Polina**
  **Cardiff**
  **South Glamorgan CF15 7NG (GB)**
- **PERNI, Stefano**
  **Cardiff**
  **South Glamorgan CF15 7NG (GB)**

(74) Representative: **Myint, Julie Marie**
**Symbiosis IP Limited**
**Basepoint Business Centre**
**Vale Business Park**
**Crab Apple Way**
**Evesham**
**Worcestershire WR11 1GP (GB)**

(56) References cited:
**WO-A1-2014/110353**
**WO-A2-2008/011561**
**US-A1- 2014 050 671**
**US-A1- 2014 193 508**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 169 367 B1

## Description

Field of the Invention

The invention relates to a complex comprising a cartilage delivery vehicle for transporting agents such as drugs, like small drug molecules, biomacromolecules or growth/maturation factors into cartilage tissue and at least one agent as described above, for use in the treatment of cartilage damage; and a method for the manufacture of said complex.

Background of the Invention

Cartilage is a tissue that lines the ends of bones and, usually but not exclusively, in combination with ligaments and muscles, constitutes the load-bearing surfaces of synovial joints and allows the frictionless movement of these joints.

Cartilage is composed of a single cell type, the chondrocyte, which is suspended at a low cell to volume ratio in an extracellular matrix that resembles a stiff hydrogel. The principal role of the chondrocyte is in the maintenance of the intricate extracellular matrix of cartilage primarily composed of proteoglycans, glycosaminoglycans and collagens. The extracellular matrix (ECM) chiefly consists of a fibrous component, mainly collagen type II, and a proteoglycan, aggrecan.

The structure of the extracellular matrix of cartilages is characterised by the presence of Glycosaminoglycans (GAGs). These are polymers made of amino sugars (N-acetylglucosamine or N-acetyl galactosamine) and uronic sugars (glucuronic acid or iduronic acid) or galactose. The resulting structure is highly negatively charged allowing cartilages to perform the required functions of shock absorber and low friction surface.

Following the chondrification that occurs during embryogenesis, cartilage growth consists mostly of the maturing of immature cartilage to a more mature state. The division of cells within cartilage occurs very slowly, and thus growth in cartilage is usually not based on an increase in size or mass of the cartilage but also remodelling. At birth, articular cartilage is morphologically distinct compared to adult tissue; immature cartilage is much thicker, chondrocytes appear to be isotropically distributed throughout the tissue with no specific zonal architecture apparent, and collagen fibrils appear to be randomly aligned. In articular cartilage it has been hypothesised that growth and, importantly, maturation proceeds through a gradual process of resorption from below and appositional growth from the surface. Articular cartilage function is dependent on the molecular composition of its extracellular matrix (ECM), which consists mainly of proteoglycans and collagens. Remodelling of cartilage is predominantly affected by changes and rearrangements of the collagen matrix, which responds to tensile and compressive forces experienced by the cartilage. Cartilage growth thus refers to matrix deposition, but can include remodelling of the extracellular matrix.

Cartilage’s structure and function can be damaged. This damage can be the result of a variety of causative factors, such as a physical traumatic injury, events precipitated by a previous injury or wear and tear over time. Immobilization for long periods can also result in cartilage damage.

Repair of injured cartilage in hips and knees is proving to be an extremely difficult principally because hyaline cartilage is avascular, aneural and consequently has a poor reparative capacity. Current surgical methods to heal cartilage defects focus on the delivery of cells embedded in a scaffold material. One of the main criticisms levelled at cell implantation techniques is that the tissue exhibits poor biomechanical performance that eventually leads to a failure of the grafted material. The poor biomechanical function of repair tissues are attributed to both inappropriate (fibrocartilagenous) differentiation and the persistence of immature cartilage tissue following surgical cellular implantation. Postnatal development of immature cartilage to a mature structured tissue can take many months, and in humans takes many years, leading some to question whether complete tissue repair is at possible. The lack and speed of tissue maturation in planted or injured articular cartilage needs to be resolved in order to provide an effective and durable treatment for patients.

Further, several diseases are known to exist that can affect cartilage. Chondrodystrophies are a group of diseases characterized by disturbance of growth and subsequent ossification of cartilage. The most common disease affecting cartilage is Rheumatoid Arthritis (RA), a chronic autoimmune disease in which skeletal joints become inflamed; and Osteoarthritis (OA), where the cartilage covering bones is thinned from "wear and tear", resulting in a "bone against bone" joint. Both diseases cause reduced motion and pain in the affected areas. Other cartilage diseases or damage include traumatic rupture or detachment, wherein cartilage is physically damaged through injury, achondroplasia, costochondritis, spinal disc herniation, and polychondritis. Tumours made up of cartilage tissue, either benign (termed chondromas) or malignant (termed chondrosarcoma), can also occur usually appearing in bone, rarely in pre-existing cartilage.

When the cartilage damage increases and the chondral defect reaches the subchondral bone, the blood supply in the bone starts a healing process in the defect. Scar tissue made up of a type of cartilage called fibrocartilage is then formed. Although fibrocartilage is able to fill in cartilage defects its structure is significantly inferior to hyaline cartilage being much denser and less able to withstand compressional force. It is therefore at a higher risk of breaking down. For

example, it is documented that small articular cartilage defects can progress to OA over time if left untreated. Indeed, this has led to the definition of a subset of the OA disease termed post traumatic osteoarthritis (PTA) i.e. degenerative joint disease secondary to injury that may lead to OA years later.

Unfortunately, cartilage has limited repair capabilities. Chondrocytes are contained in cavities in the matrix, called cartilage lacunae; this constitutes the so-called capsule of the space. Because chondrocytes are bound in lacunae, they cannot migrate to damaged areas and as such cartilage damage is difficult to heal. Additionally, unlike other connective tissues cartilage is avascular, aneural and contains no lymphatic vessels it also has low metabolic activity. These latter characteristics and the fact that the tissue is composed of a highly hydrated ECM impede repair and/or regeneration following trauma or disease. Thus, compared to other connective tissues, cartilage grows and repairs more slowly or not at all.

Though cartilage damage is not life threatening, it can severely affect the quality of life.

Recent research has focussed on regenerating damaged joints, or more ideally preventing degeneration in the first place. Numerous disease-modifying anti-rheumatic drugs (DMARDS) for rheumatoid arthritis and several related rheumatic diseases are available (such as methatrexate and tumour necrosis factor alpha blockers (TNF$\alpha$ blockers)); conversely no effective disease modifying osteoarthritis drugs (DMOADS) are currently available. At present, therapies for OA are capable of providing only short term relief of pain and inflammation are offered; these can be through the administration of steroidal or non-steroidal anti-inflammatory drugs (NSAID).

Alternatively, tissue engineering, the generation of biofunctional and biocompatible tissue for therapeutic regeneration and repair, has advanced to the stage where it is possible to generate artificial cartilage constructs that can be either pre-conditioned and grown *in vitro* prior to implantation, or, designed to fulfil the latter objectives *in vivo* following implantation and exposure to suitable agents such as growth factors and the like.

Unfortunately, many drugs used to treat cartilage damage, including arthritis, have serious side effects, e.g. dexamethasone, shown to have positive effects for the treatment of inflammatory and autoimmunity diseases, has been linked to bone loss, muscle weakness and atrophy, suppression of the adrenal gland, increased risk of infection, peptic ulcer disease and growth retardation. Dexamethasone can also cause problems with vision; swelling, rapid weight gain, feeling short of breath; severe depression, unusual thoughts or behaviour, seizure (convulsions); bloody or tarry stools, coughing up blood; pancreatitis; muscle weakness; high blood pressure, insomnia and skin problems (acne, dry skin, thinning skin, bruising or discoloration);

Prednisolone can cause effects including muscle pain/cramps, irregular heartbeat, weakness, swelling hands/ankles/feet, unusual weight gain, signs of infection (such as fever, persistent sore throat), vision problems (such as blurred vision), vomit that looks like coffee grounds, black/bloody stools, severe stomach/abdominal pain, mental/mood changes (such as depression, mood swings, agitation), slow wound healing, thinning skin, bone pain, menstrual period changes, puffy face, seizures, easy bruising/bleeding. It may also increase blood sugar level, which can cause or worsen diabetes.

It is thought these effects could be mitigated or overcome if the drug was delivered to, or constrained within, the cartilage tissue. Nanoparticles, in particular based on avidin/biotin-conjugates, for enabling drug penetration into the deeper zones of cartilage are disclosed in US2014/0193508 A1.

Hence there is an unmet need for the targeted delivery of drugs used to treat or alleviate cartilage damage and/or promote repair. Moreover there is also an unmet need for the targeted delivery of factors used to promote cartilage development/repair, particularly in tissue engineering interventions.

We describe herein a delivery vehicle for delivering agents into cartilage thus improving drug efficacy at lower concentrations, enhancing agent retention in the cartilage, reducing the side effects of the agents and reducing treatment costs.

## Statements of Invention

The applicants have found that poly($\beta$-amino esters (PBAEs) or derivatives thereof are able to penetrate into cartilage tissue and act as carriers for agents in particular therapeutic agents such as drugs, like small drug molecules, biomacromolecules or growth factors. Without being bound by theory, it appears that this may be due to the electrostatic attraction between the negatively charged components of the cartilages extracellular matrix and the positively charged PBAEs.

According to a first aspect of the invention there is provided a polymer comprising poly($\beta$-amino ester (PBAE) or a PBAE derivative having a functional group at the end of the polymer chain, and a therapeutic agent effective in the treatment of a cartilage disease or condition, for use in the treatment of cartilage damage, wherein the PBAE or PBAE derivative is covalently bound to the therapeutic agent and said complex is administered such that the complex penetrates pre-existing cartilage tissue. The polymer is able to penetrate cartilage cells, even when combined with a therapeutic agent. In order to achieve this, the polymer will not be subject to procedures such as cross-linking, to produce rigid three-dimensional structures.

PBAEs have been found to have good biocompatibility and solubility as compared to other available polycations such as poly-L-lysine and polyethylenimine (PEI). Furthermore, they have shown good cytocompatibility *in vitro.*
As used herein, the expression poly(β-amino ester) or PBAE refers to a polymer obtainable by reaction of a diacrylate monomer with an amine monomer. Suitable diacrylate monomers are compounds of formula (I)

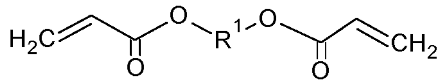

(I)

where $R^1$ is an optionally substituted divalent hydrocarbyl group, which may also be interposed with heteroatoms, with a primary or secondary amine monomer.
As used herein, the expression 'hydrocarbyl' refers to groups made up of carbon and hydrogen atoms. These include alkyl, alkenyl or alkynyl groups, as well as aromatic groups such as phenyl or combinations of these. For example, hydrocarbyl groups may comprise carbon chains which are substituted with or interposed with aromatic groups such as phenyl. Divalent forms include alkylene, alkenylene, alkynylene etc. Alkyl groups may be straight or branched chains, or they may form or include cycloalkyl rings. Hydrocarbyl groups may suitably comprise from 1-20 carbons atoms, for example from 2-10 carbon atoms. Alkyl groups may comprise 1-10 carbon atoms such as from 1-6 carbon atoms, whilst alkenyl and alkynyl groups suitably comprise from 2-10 such as from 2-6 carbon atoms.
The expression 'heteroatom' refers to an atom which is not a carbon atom, such as oxygen, nitrogen, sulphur or silicon. The expression 'heterocyclic' or 'heterocyclyl' refers to groups which comprise rings suitably of from 3-20 atoms, at least one of which is a heteroatom. The rings may be monocyclic or fused ring systems and they may be aromatic of non-aromatic. Examples of such groups include pyridyl, pyrimidyl, pyrrolyl, thiophenyl, furanyl, indolyl, quinolyl, isoquinolyl, imidazoyl and triazoyl groups.
The term 'heteroaryl' refers to such ring systems which are aromatic in nature such as pyridyl.
In particular $R^1$ is an optionally substituted alkylene chain which are optionally interposed with one or more heteroatoms, in particular oxygen atoms.
Where $R^1$ is an alkylene chain interposed with oxygen atoms, it may comprise an ethylene glycol chain. For example, the compound of formula (I) may comprise tetra(ethylene glycol) diacrylate.
Suitable substituents for $R^1$ groups include halo groups such as fluoro or chloro and well as hydroxyl groups.
In particular however, $R^1$ is a $C_{1-10}$alkylene chain, for instance $C_{1-6}$alkylene. In a particular embodiment, $R^1$ is an n-butylene group or n-hexylene. Thus particular compounds of formula (I) are 1,4-butanediol diacrylate or 1,6-hexanediol diacrylate.
Suitable amine monomers include primary amines or secondary diamines such as those of formula (II) or (III)

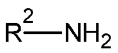

(II)

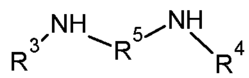

(III)

where $R^2$ is an optionally substituted hydrocarbyl group;
$R^3$ and $R^4$ are independently selected from optionally substituted hydrocarbyl groups;
$R^5$ is a divalent hydrocarbyl group which is optionally substituted and may also be interposed with heteroatoms such as oxygen;
or $R^3$ and/or $R^4$ are linked together or to $R^5$ to form one or more ring structures.

Suitable optional substitutents for the hydrocarbyl groups $R^3$, $R^4$ and $R^5$ comprise one or more groups selected from hydroxyl, $C_{1-6}$alkoxy such as methoxy, $C_{1-6}$alkylsilane or heterocyclic groups or halo.
Suitable optional substituents for $R^2$ are one or more groups selected from hydroxyl, $C_{1-6}$alkoxy such as methoxy, $C_{1-6}$alkylsilane or heteroaryl groups or halo.
Suitable, $R^2$ is an optionally substituted alkyl group, in particular an optionally substituted $C_{1-6}$alkyl group wherein the optional substituents are as described above.
Suitably, $R^3$ or $R^4$ are optionally substituted alkyl groups, in particular unsubstituted $C_{1-6}$alkyl such as methyl.
In a particular embodiment $R^3$ and $R^4$ are linked together to form a ring structure, in particular a 5-6-membered nitrogen containing ring such as a piperazine ring. In another particular embodiment, $R^3$ and/or $R^4$ are linked to $R^5$ to form a piperidine ring.

Suitably $R^5$ is an optionally substituted alkylene chain, in particular an unsubstituted $C_{1-10}$alkylene chain, such as ethylene.

Particular examples of monomers of formula (II) include 5-amino-1-pentanol, 2-methoxyethylamine, 3-(dimethylamino)-propylamine, (3-aminopropyl) triethoxysilane, 2-(2-pyridyl)ethylamine, 3-methoxy-propylamine, 3-amino-1,2-propanediol, 3-methoxypropylamine, 4-(2-aminoethyl)morpholine, cyclopentylamine, 4-amino-hexanol and 1-amino-2 propanol.

For instance monomers of formula (II) may be 5-amino-1-pentanol, 2-methoxyethylamine, 3-(dimethylamino)-propylamine, (3-aminopropyl) triethoxysilane, 2-(2-pyridyl)ethylamine, 3-methoxy-propylamine, 3-amino-1,2-propanediol and 1-amino-2 propanol.

Particular examples of monomers of formula (III) include piperazine, 4,4-trimethylenedipiperidine and 3-(dimethylamino)-propylamine.

In a particular embodiment, the amine monomer is a compound of formula (III) or is a compound of formula (II) wherein $R^2$ carries at least one substituent which includes a nitrogen atom and /or is a hydroxyl group. Examples of such compounds are 4,4-trimethylenedipiperidine, 3-(dimethylamino)-propylamine, 2-(2-pyridyl)ethylamine and 1-amino-2 propanol.

PBAEs including such substituents appear to be particularly effective vehicles carrying agents into cartilage tissue. Again, without being bound by theory, it is possible that the monomers comprising additional nitrogen produce an increased positive charge in the polymer chain, which may increase the electrostatic attraction towards the negatively charged biopolymers constituting the extracellular matrix of cartilage.

If the PBAEs produced are required to have a vinyl terminal group, the acrylate monomer needs to be present in excess. However, the greater the excess the shorter the PBAE chain produced. Although a smaller chain would allow a higher percentage of drug to be present in the final formulation, if the chain is too short it is unlikely to be effective as it would lack sufficient charge. Thus the acrylate monomer is suitably present in a small excess as compared to the amine monomer. Typically, the ratio of diacrylate monomer to amine monomer in the PBAE is in the range of from 2:1 to 1.05:1, such as 1.1:1.

The length of the PBAE polymer is such that the average molecular weight is in the range of from 4,000-40,000, for example from 12000-30000 such as from 18000-20000.

Examples of PBAEs as described above may be represented by formula (IV) and (V)

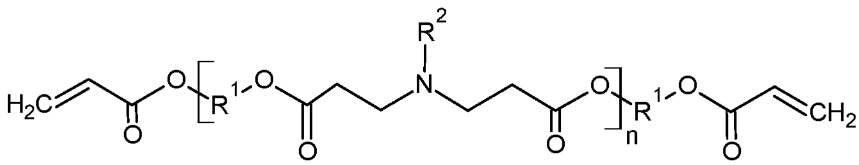

(IV)

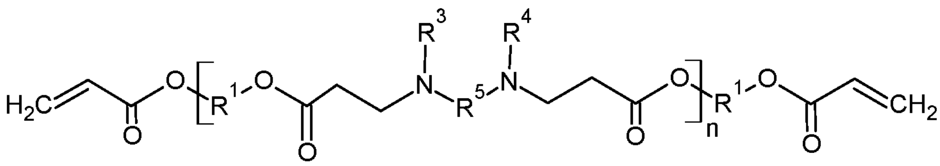

(V)

where $R^1$ is as defined above in relation to formula (I), $R^2$ is as defined above in relation to formula (II) and $R^3$, $R^4$ and $R^5$ are as defined above in relation to formula (III); and n is a integer greater than 2, in particular in the range of from 10-100, for example from 30-70 such as from 45-55.

In the complex of the invention, the agent is covalently bound to the ends of the PBAE chains. In particular, a PBAE derivative may be formed which is then reacted with the agent to form a covalent bond.

Derivatives of the PBAEs have a functional group at an end of the polymer chain. Such derivatives for use in the treatment of form a particular embodiment of the invention.

As used herein, the expression 'functional group' refers to groups which are reactive to allow the formation of covalent bonds to agents, in particular pharmaceutical agents, which may themselves be modified by addition of a functional group able to react with the functional group present on the PBAE.

In a particular embodiment, the functional group is such as to be able to allow an amide bond to be formed

between the agent and the PBAE. In such cases, one of the functional groups on the agent or the PBAE comprises a carboxylic acid or acid salt and the functional group on the other of the agent or the PBAE comprises a primary or secondary amino group.

In a particular embodiment, the PBAE derivative carries amino functional groups at the ends of the polymer chains forming 'end-caps'.

Amino functional groups may comprise primary or secondary amino groups, able to link to an agent. Such functional groups or end caps may be introduced by reacting the PBAE with a primary or secondary diamine compound, for example a primary diamine of formula (VI)

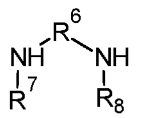
(VI)

where $R^6$ is an alkylene chain, and
$R^7$ and $R^8$ are the same and are selected from hydrogen or a $C_{1-6}$alkyl group.

In a particular embodiment, $R^6$ is a $C_{2-6}$alkylene chain which is optionally interposed with a heteroatom, in particular a nitrogen atom. The nitrogen atom may be substituted with an optionally substituted $C_{1-6}$alkyl group, such as an alkyl amino group, for example 2-aminoethyl.

In particular, $R^7$ and $R^8$ are both hydrogen.

Particular examples of compounds of formula (VI) are ethylene diamine and diethylenetriamine. Thus particular examples of $R^6$ are ethylene and diethyleneamino. In a particular embodiment, the compound of formula (VI) is diethylenetriamine. In a further embodiment, the compound of formula (VI) is tris-(2-aminoethyl)amine.

Thus suitable PBAE derivatives of the invention may be represented by formula (VII) or (VIII)

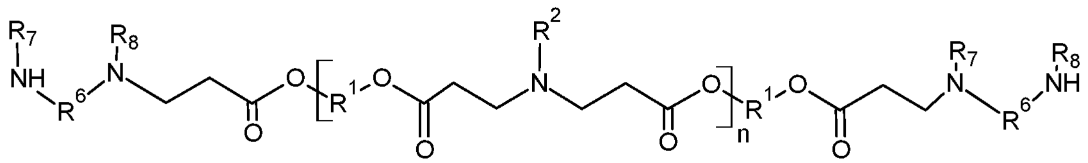
(VII)

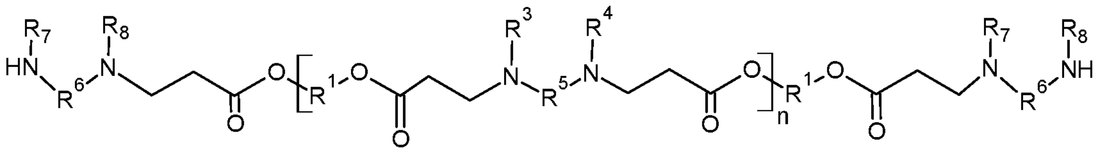
(VIII)

wherein $R^1$, $R^2$ $R^3$, $R^4$, $R^5$, $R^6$, $R^7$, $R^8$ and n are as defined above.

Particular groups $R^1$, $R^2$ $R^3$, $R^4$, $R^5$, $R^6$ and n are also as defined above.

In an alternative embodiment, the functional group is such as to be able to allow an ester bond to be formed between the agent and the PBAE. In such cases, one of the functional groups on the agent or the PBAE comprises a carboxylic acid or acid salt and the functional group on the other of the agent or the PBAE comprises an alcohol group.

In a particular embodiment, the PBAE derivative carries an alcohol functional group at the ends of the polymer chains forming 'end-caps' that may react with carboxylic acid functionalities on agents. They may also react with hydroxyl groups on drugs if a suitable linker such as suberic acid is included in the reaction.

Such functional groups or end caps may be introduced by reacting the PBAE with an amino alcohol or mercapto-alcohol instead of a primary or secondary diamine compound, for example an alcohol formula (VIA)

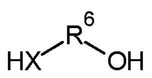
(VIA)

where $R^6$ is as defined above, and X is NH or sulfur.

In particular, in these compounds, $R^6$ is an unsubstituted alkylene group. Particular examples of compounds of formula (VIA) are 2-aminoethanol, 3-amino-1-propanol, 4-amino-1-butanol, 5-amino-1-pentanol and 2-mercaptoethanol.

Thus suitable PBAE derivatives of the invention may be represented by formula (VIIA) or (VIIIA)

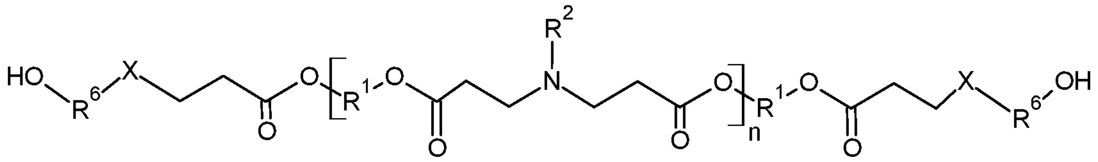

(VIIA)

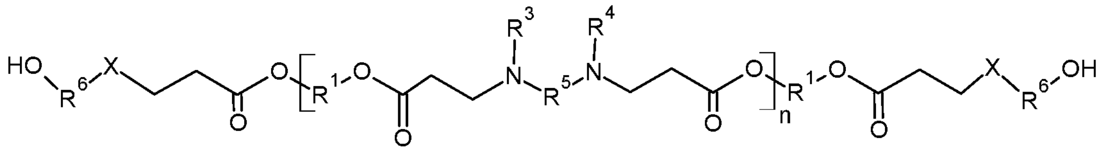

(VIIIA)

wherein $R^1$, $R^2$ $R^3$, $R^4$ and $R^5$ are as defined above,
$R^6$ is an alkylene chain which is optionally interposed with a heteroatom, which may be substituted,
n is an integer of greater than 2, and X is NH or sulfur.

Derivatives of a PBAE as described above may be prepared by reacting a PBAE with a compound of formula (VI) or (VIA) as defined above. The compound of formula (VI) or (VIA) is suitably present in excess. The reaction is suitably carried out in an organic solvent such as dichloromethane (DCM) or tetrahydrofuran (THF) at moderate temperatures, for example from 10-50°C, and conveniently at ambient temperature. The reaction mixture may be retained under these conditions for a sufficient period to ensure that end-capping is substantially complete, typically from 10-36 hours for example about 24 hours. Thereafter, the PBAE derivative may be recovered by precipitation into an antisolvent such as diethyl-ether. Excess compound of formula (VI) or (VIA) may then be removed for example by centrifugation, and the resulting derivative may be isolated.

The PBAE starting material may be prepared using methods known in the art, for example as described in Anderson et al. Angew. Chem. Int. Ed. 2003, 42, 3153-3158. In particular, they may be prepared by mixing together a monomer of formula (I) above with an appropriate amount of an amine monomer such as those of formula (II) or (III) above to achieve the desired polymer. The reaction is suitably carried out in an organic solvent such as DCM, at elevated temperatures for example of from 30-80°C, for example at about 50°C, with agitation. Polymer may be recovered by precipitation using an antisolvent such as diethylether.

In some instances, PBAE derivatives having hydroxyl end groups instead of acrylate end groups may be prepared directly, by reacting the monomer of formula (I) above with an excess of an amine monomer, which in this case, may be of formula (VIA) as described above.

PBAE derivatives such as those of formula (VII) or (VIIA) or formula (VIII) or (VIIIA) may be readily covalently linked to agents which have, or which are modified to include carboxylic acid groups.

Suitable agents for use in the complexes of this aspect of the invention will comprise therapeutic agents or substances that have a beneficial effect in cartilage. They may include drugs in particular small drug molecules as well as biomacromolecules, biological agents including growth/maturation factors.

Suitable drugs include those used in the treatment of arthritis including osteoarthritis and rheumatoid arthritis. Such drugs include steroidal anti-inflammatory drugs such as dexamethasone, prednisolone, 6α-methylprednisolone; DMARDS such as methotrexate, sulfasalazine; non-steroidal anti-inflammatory drugs (NSAID) such as ibruprofen, naproxen, ketorolac tromethamine, Celecoxib and indomethacin or AMPA receptor antagonists such as 2,3-dihydroxy-6-nitro-7-sulfamoyl-benzo[f]quinoxaline-2,3-dione (NBQX).

In particular, the drug is selected from dexamethasone, 6α-methylprednisolone, methotrexate, ketorolac tromethamine, sulfasalazine, indomethacin and NBQX (AMPA receptor antagonists).

Steroidal drugs (such as Dexamethasone and Prednisolone) that present hydroxyl group are functionalised with succinic anhydride as illustrated in the following schemes, resulting in an ester bond formation between the drug and the linker (succinic anhydride); the functionalised drug exhibits a carboxyl group.

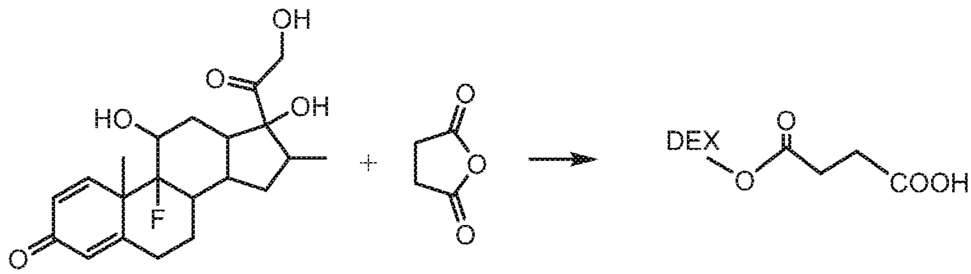

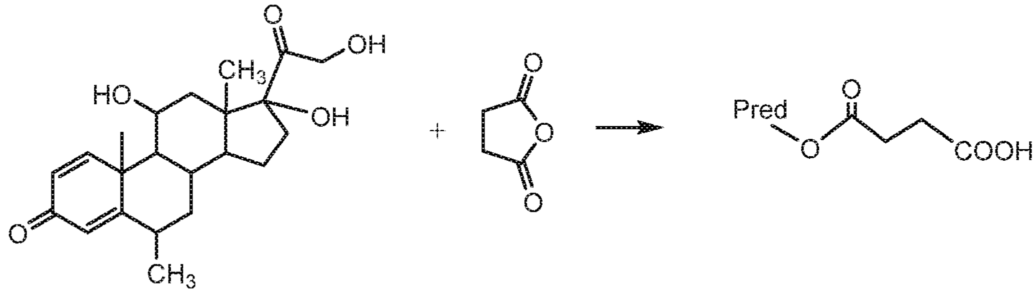

The terminal carboxyl group may be is employed in the conjugation to the end-capped PBAE as illustrated below. Since the resultant ester bond is hydrolysable it may be utilised to achieve drug release from the PBAE carrier.

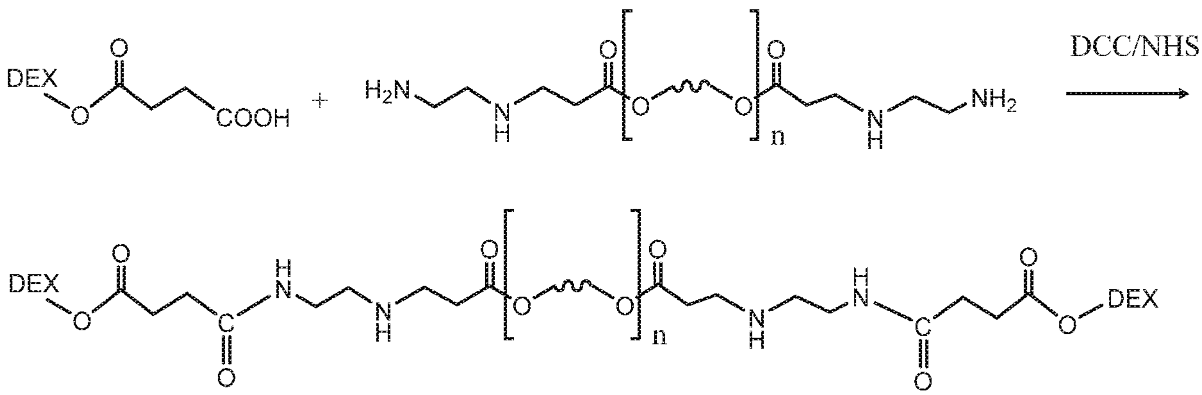


In the above schemes, the wavy lines represent the repeat unit of the polymeric elements such as those represented above.

Biomacromolecules include biological macromolecules that may be used to treat cartilage conditions such as glycosaminoglycans, hyaluronic acid (HA) and hormones, for instance calcitonin.

Growth factors are growth promoting molecules that are classified in large families of structurally and evolutionarily related proteins. These families are listed as follows: Adrenomedullin (AM), Autocrine motility factor, Bone morphogenetic proteins (BMPs), Epidermal growth factor (EGF), Erythropoietin (EPO), Fibroblast growth factor (FGF), Granulocyte-colony stimulating factor (G-CSF), Granulocyte-macrophage colony stimulating factor (GM-CSF), Growth differentiation factor-9 (GDF9), Hepatocyte growth factor (HGF), Hepatoma derived growth factor (HDGF), Insulin-like growth factor (IGF), migration- stimulating factor, Myostatin (GDF-8), Nerve growth factor (NGF) and other neurotrophins, Platelet-derived growth factor (PDGF), Thrombopoietin (TPO), Transforming growth factor alpha(TGF-$\alpha$), Transforming growth factor beta (TGF-$\beta$), Vascular endothelial growth factor (VEGF), placental growth factor (PIGF), Foetal Bovine Somatotrophin (FBS), Heparin binding (HB), Heparin-binding like EGF growth factors (HB-EGF), IL-1-Cofactor for IL-3 and IL-6, IL-2- T-cell growth factor, IL-3, IL-4, IL-5, IL-6, and Il-7.

Growth factors have a role to play in both cartilage growth and maturation. The former being characterised by an increase in cell mass or ECM mass and the latter by a change in the morphogenetic characteristics of the tissue as explained in some detail above. Typically, growth factors act in an anabolic fashion promoting cartilage matrix synthesis leading to chondrocytes being surrounded by abundant matrix. An example of a growth factor that has this effect is FGF-18. Other growth factors, such as TGF-alpha or TGF-beta have also been shown to have anabolic and homeostatic

effects resulting in a regulated increase in glycosaminoglycan and collagen content of the ECM leading to growth of the hyaline cartilage.

However, FGF has also been shown to inhibit chondrocyte proliferation by initiating multiple pathways that result in the induction of antiproliferative functions. Further, a cocktail of growth factors i.e. IGF-I, FGF- 2 (also known as FGF-basic or bFGF), and TGF-betal have been shown to regulate their own and each other's activities, their interactions ranging from inhibitory to synergistic effects. These studies suggest that interactions among IGF-I, FGF-2, and TGF-betal substantially modulate their regulatory functions.

Although the role of growth factors in the growth or maturation process of cartilage is not clearly defined, it may be of clinical benefit to supply one or more of such factors in therapy. In such cases, they may be administered in combination with the vehicles of the invention.

The ability to artificially mature cartilage *in-vitro* thus mimicking the natural process *in vivo* has been shown (Khan et al. Arthritis and Rheumatism (2011) 63 (11):3417-3427) and evidence provided that growth factors fibroblast growth factor-2 (FGF2) and transforming growth factor beta-1 (TGF$\beta$1) work combinatorially to highly accelerate postnatal maturation of immature articular cartilage, even in the absence of biomechanical input. Furthermore, it has been demonstrated that immature cartilage that had been 'matured' *in vitro* using growth factors had the same fundamental properties: frictional coefficient, stiffness, surface roughness and biochemical profile as native (*in-vivo*) mature articular cartilage (Khan et al. Biomaterials (2013) 34(5):1478-1487). TGF-$\beta$1 stabilizes the synthesis of cartilaginous matrix (PG and type II collagen) by chondrocytes: it promotes tissue homeostasis with no overall change in their size, composition or biomechanical function. FGF-2 exerts opposing effects on immature cartilage, promoting mitosis and anabolism at low concentration (<30 ng/ml) but inhibiting the same processes at higher (30-300 ng/ml) concentration. Combinations of factors such as FGF-2 and TGF-$\beta$1 have synergistic effects on chondrocytes, repressing collagen type II gene expression, reducing the doubling time of culture expanded human chondrocytes and maintaining their phenotypic stability.

In a particular embodiment however, the agent is a drug as described above.

Where necessary these drugs may be derivatised to facilitate covalent attachment to the PBAE derivative as described above. In particular, the drugs may be succinylated, for example by reaction with succinic anhydride to convert a hydroxyl group in the drug structure to a succinic acid group. The reaction conditions will depend upon the particular drug involved. However, typically, the reaction may be carried out in a solvent such as dimethyl-formamide (DMF) at moderate temperatures for example of from 10-50°C and conveniently at ambient temperature. The reaction may suitably be carried out under an inert atmosphere for example a nitrogen atmosphere. The succinic acid group introduced in this way will then readily form a covalent bond with a PBAE derivative having an amine end-cap, such as a derivative of formula (VII) or (VIII) as described above, to form the desired complex.

In a second aspect, the invention provides a method for preparing a complex as described above, which method comprises reacting together a PBAE derivative as described above with an agent or a derivative thereof.

In a particular embodiment, the method of the invention involves 4 steps which are summarized in Figure 1 hereinafter. In particular, the polymer is first synthesised from a mixture of acrylate and amine with acrylate excess (based on molar ratio) in order to achieve a polymer with acrylate ends as illustrated hereinafter in Example 1. The acrylate terminated PBAE are then reacted with end-capping agents exhibiting either amino or mercapto groups and a further amino or hydroxyl group as illustrated in Example 2 hereinafter. In case of di-amine compounds these are symmetric. At the end of this reaction, the PBAEs exhibit either an amino or a hydroxyl group at the extremities providing moieties for the conjugation of the drug.

The reaction conditions employed with vary depending upon factors such as the particular nature of the vehicle or linker and the agent or whether it is derivatised. These will be determined by a chemist using their skill and knowledge. For example, a reaction between a succinylated agent and an amine end-capped PBAE such as a derivative of formula (VII) or (VIII), the reaction is suitably carried out in an organic solvent such as DCM, in the presence of a carboxylic activating agents such as N,N'-Dicyclohexylcarbodiimide (DDC) and N-hydroxysuccinimide (NHS). The reaction may be carried out at moderate temperatures for example of from 10-50°C and conveniently at room temperature. The complexes may be precipitated out of solution using an anti-solvent such as diethyl-ether and then recovered for example by evaporation of the solvents.

The complexes of the invention may be used in therapy, in particular for the treatment of conditions or diseases that are associated with or related to cartilage tissue. Such conditions include reduction in cartilage tissue due to wear and tear, as well as diseases including arthritis including rheumatoid arthritis, osteoarthritis ankylosing spondilitisis, tendonitis or traumatic injury.

For use in these therapies, the vehicles are combined with a therapeutic agent as described above, in particular in the form of a complex as described above and are suitably administered in the form of a pharmaceutical composition.

Thus a third aspect of the invention provides a pharmaceutical composition comprising a vehicle and an agent as described above, in particular in the form of a complex also, as described above in combination with a pharmaceutically acceptable carrier for use in the treatment of cartilage damage.

Suitable pharmaceutical compositions will be in either solid or liquid form. They may be adapted for administration

by any convenient route, but in particular will be by parenteral administration. The pharmaceutical acceptable carrier may include diluents or excipients which are physiologically tolerable and compatible with the active ingredient. These include those described for example in Remington's Pharmaceutical Sciences, Mack Publishing Co. (A.R. Gennaro edit. 1985).

Parenteral compositions are prepared for injection, for example either intra-articularly, subcutaneously, intramuscularly, intradermally, intravenously or via needle-free injection systems. In particular, the compositions will be administered by the intra-articular route. They may be liquid solutions or suspensions, or they may be in the form of a solid that is suitable for solution in, or suspension in, liquid prior to injection. Suitable diluents and excipients are, for example, water, saline, dextrose, glycerol, or other pharmaceutially acceptable organic solvents such as ethanol, and combinations thereof. In addition, if desired the compositions may contain minor amounts of auxiliary substances such as wetting or emulsifying agents, stabilizing or pH-buffering agents.

The amount of therapeutic agent and PBAE or derivative to be administered will vary depending upon factors such as the specific nature of the agent used, the size and health of the patient, the nature of the condition being treated etc. in accordance with normal clinical practice. Typically, a dosage in the range of from 0.001-1000 mg/Kg, for instance from 0.1-10 mg/Kg, would produce a suitable therapeutic effect.

Dosages may be given in single dose regimes, split dose regimes and/or in multiple dose regimes lasting over several days. Effective daily doses will, however vary depending upon the inherent activity of the therapeutic agent, such variations being within the skill and judgment of the physician.

The vehicle/agent combinations of the present invention may be used in combination with one or more other active agents, such as one or more pharmaceutically active agents.

PBAEs possess positive charges because of the presence of amine groups; however the net charge can vary depending on the structure of the acrylates and amine; the PBAE prepared using amino-alcohols exhibited lower zeta potentials because OH can be deprotonated resulting in a negative charge; whilst the presence of additional nitrogen atoms have been found to return a higher zeta potential as additional positive charge can be formed.

Trypsine is used to reduce the quantity of GAG presents in cartilage and such mimics the situation in osteoarthritis. PBAEs rely on their electrostatic attraction towards the negatively charged GAG to enhance drug uptake; therefore a reduction in GAG could have resulted in lower efficacy as drug delivery system in situations (cartilage affected by OA) when drugs would have been used. The applicants have found that despite the reduction of GAG content the uptake of a drug such as DEX was greater than in case of cartilage with normal amount of GAG. Such circumstance is likely to depend on the reduced steric inherence exhibited by GAG depleted cartilage to the penetration of PBAE. Hence, PBAE are an effective drug delivery system for cartilages even when the amount of GAG is severely limited.

Drug "wash out" from cartilages is known to reduce efficacy so it is important that not only uptake but also retention is improved. The applications have found that a drug such as DEX is retained in cartilages through the use of PBAE-DEX for longer period of time that when a simple DEX phosphate solution is used.

As exemplified hereinafter, viability of chondrocytes was not affected by the presence of PBAE in the media and this is essential for medical application. PBAEs are known to be cytocompatible but this is the first time that such property (linked to easy hydrolysation and biological compatibility of the degradation products) is shown in chondrocytes).

End capping is the result of the reaction between a nucleophile and a carbon double bonds in position alfa-beta from a carbonyl group (Michael reaction or Michael addition). Primary amine and thiols can undergo such reaction but hydroxyl group are not generally susceptible. For these reasons the applicants carried out end-capping with symmetric di-amines or amino-alcohols to allow the formation of the bond between acrylates and amino groups, in the same way thio-alcohols allowed the formation of bond between the acrylate and sulphur. The length of the chain had an effect on the drug delivery efficacy of the PBEA end-capped in this way as with compounds too long or too short having the worst performance.

The efficacy of PBAE to delivery prednisolone was higher than dexamethasone probably in virtue of the higher hydrophilicity of Prednisolone.

The applicants have also found that conjugation between PBAE and drug is essential for the drug delivery performance, as the electrostatic attraction between dexamethasone and PBAE was not enough to allow the delivery of the steroid into cartilages. In some cases the presence of unconjugated PBAE was detrimental to the overall uptake of DEX, likely the presence of diffusing pure PBAE molecules obstacles the penetration of pure DEX molecules.

Throughout the description and claims of this specification, the words "comprise" and "contain" and variations of the words, for example "comprising" and "comprises", mean "including but not limited to" and do not exclude other moieties, additives, components, integers or steps. Throughout the description and claims of this specification, the singular encompasses the plural unless the context otherwise requires. In particular, where the indefinite article is used, the specification is to be understood as contemplating plurality as well as singularity, unless the context requires otherwise.

No admission is made that any reference cited in this specification constitutes prior art. Further, no admission is made that any of the prior art constitutes part of the common general knowledge in the art.

Preferred features of each aspect of the invention may be as described in connection with any of the other

aspects.

Other features of the present invention will become apparent from the following examples. Generally speaking, the invention extends to any novel one, or any novel combination, of the features disclosed in this specification (including the accompanying claims and drawings). Thus, features, integers, characteristics, compounds or chemical moieties described in conjunction with a particular aspect, embodiment or example of the invention are to be understood to be applicable to any other aspect, embodiment or example described herein, unless incompatible therewith. Moreover, unless stated otherwise, any feature disclosed herein may be replaced by an alternative feature serving the same or a similar purpose.

The invention will now be described by way of example only with reference to the Examples below and to the following Figures wherein:

Figure 1(A) is a schematic diagram showing a general method of synthesis of the polymers of the invention (B) is a schematic of an alternative method which avoids functionalization of the drug; (C) is a schematic of an alternative method in which the drug complexes are prepared in three steps; and (D) illustrates a two-steps method.

Figure 2 is a schematic diagram of a transport chamber (a) prepared for use in experiments showing the efficacy of complexes of the invention in cartilage tissues, and (b) illustrates the flow through the cartilages sample;

Figure 3 is a graph showing the non-equilibrium diffusive transport of PBAE-FITC across a 6 mm diameter, ~400 mm thick cartilage explants, plotted as the ratio between measured downstream and upstream concentration versus time where ● is the PBAE represented by A1-e1 and ○ is the PBAE represented by A2-e2.

Figure 4 is a graph showing a comparison of DEX uptake in normal cartilages (a) and GAG depleted cartilages (b) using PBAE-DEX; where ■ represents A1-e1-DEX, ▼ represents A2-e1-DEX and ○ represents DEX-P.

Figure 5 is a graph showing the results of an investigation into the viability of chondrocytes exposed to DEX-P (black columns), A1-e1-DEX (grey columns) and A2-e2- DEX (white columns) assessed through MTT (a) and (LDH) assay.

Figure 6 shows the results of cartilage uptake of DEX using PBAE-DEX after 10 min exposure compared to DEX-P for a range of complexes.

Figure 7 are graphs showing correlations between DEX uptake PBAE-DEX end-capped with e1 and $\mathfrak{D}_{eff}$ (a) and zeta potential (b) of PBAEs.

Figure 8 is a graph showing correlations between zeta potential and $\mathfrak{D}_{eff}$ of PBAEs.

Figure 9 is a graph showing dexamethasone uptake in cartilage using PBAE-DEX prepared through conjugation of succinylated Dexamethasone to PBAE end capped with amino-alcohols or thio-alcohols. (black columns for A5 and grey columns for A12).

Figure 10 is a graph showing cartilage uptake of 6α-methylprednisole using PBAE-Pred after 10 minutes exposure compared to Prednisolone acetate.

Figure 11 is a graph showing a comparison of DEX retention in normal cartilages (a) and GAG depleted cartilages (b) using PBAE-DEX; where ■ represents A1-e1-DEX, ▼ represents A2-e2-DEX and ○ represents DEX-P.

Figure 12 is a graph showing cartilage uptake of DEX when in solution pure or mixed with various unconjugated PBAEs where ● represents DEX-P, ○ represents A1-e1, ▼ represents A2-e1 and Δ represents A5-e1.

Figure 13 is a graph showing Ketorolac uptake in cartilage using PBAE-Ket prepared through direct conjugation to PBAE end capped with amino-alcohols or thio-alcohols (black columns for A5 and grey columns for A12).

Figure 14 is a graph showing Indomethacin uptake in cartilage using A5-Ind prepared through direct conjugation to PBAE end capped with amino-alcohols or thio-alcohols.

Figure 15 shows Sulfasalazine uptake in cartilage using A5-Sulfa prepared through direct conjugation to PBAE end capped with amino-alcohols or thio-alcohols.

Figure 16 shows DEX uptake in cartilage using PBAE-DEX prepared through direct conjugation of Dexamethasone to PBAE. (black columns for PBAE end-capped with e1 and gray columns for PBAE end-capped with e2).

Figure 17 shows 6α-methylprednisole uptake in cartilage using PBAE-DEX prepared through direct conjugation of 6α-methyl-Prednisolone to PBAE. (black columns for PBAE end-capped with e1 and gray columns for PBAE end-capped with e2).

Figure 18 shows DEX uptake in cartilage using PBAE-DEX prepared through conjugation of Dexamethasone functionalised with aminoacid to acrylate terminated PBAE. (black columns for BOC-glycine and gray columns for BOC-β-alanine).

Figure 19 shows drug uptake in cartilage using PBAE-drug prepared through two steps synthesis protocol. (black columns for A16 and gray columns for B16).

Figure 20 shows the mean value of the magnitude (top) and phase angle (bottom) of the dynamic modulus, |*G**|, versus frequency of cartilages maturated without growth factors; where ● is at 0 days, ○ is at 3 days, ▼ is at 1 week, Δ is at 2 weeks and ■ 3 is at weeks.

Figure 21 shows shows the mean value of the magnitude (top) and phase angle (bottom) of the dynamic modulus, |*G**|, versus frequency of cartilages maturated with growth factors; where ● is at 0 days, ○ is at 3 days, ▼ is at 1 week, Δ is at 2 weeks and ■ 3 is at weeks.

Figure 22 shows the mean value of the magnitude (top) and phase angle (bottom) of the dynamic modulus, |*G**|, versus frequency of cartilages maturated with growth factors conjugated to A2-e1; where ● is at 0 days, ○ is at 3 days, ▼ is at 1 week, Δ is at 2 weeks and ■ 3 is at weeks.

Figure 23 shows the mean value of the magnitude (top) and phase angle (bottom) of the dynamic modulus, |*G**|, versus frequency of cartilages maturated with growth factors conjugated to A5-e2 ; where ● is at 0 days, ○ is at 3 days, ▼ is at 1 week, Δ is at 2 weeks and ■ 3 is at weeks.

Figure 24 shows box whiskers plots of the friction coefficient of cartilages matured in vitro under various conditions including with and without growth factors and with growth factors conjugated to A2-e1 and A5-e2.

Example 1

Preparation of PBAE

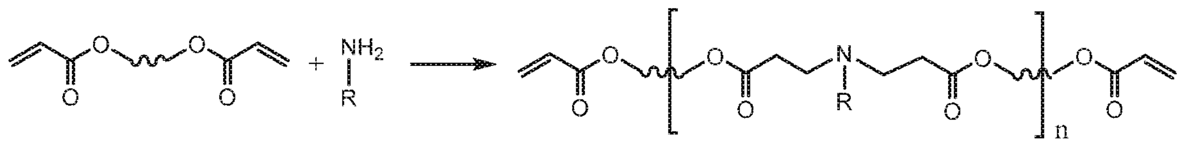

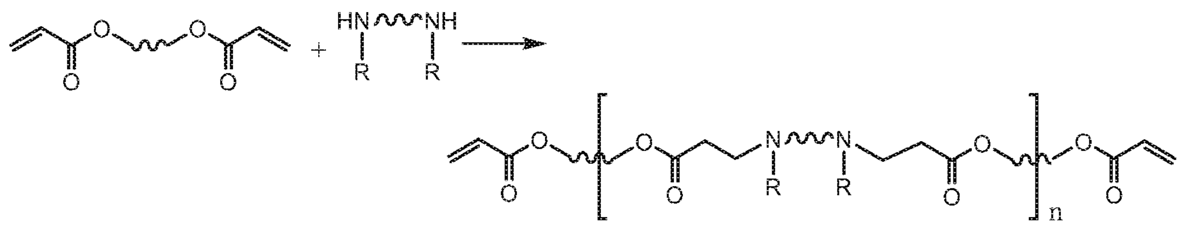

Acrylate-terminated poly(*α*-amino ester)s were synthesized by mixing 1,4-butandiole diacrylate (A), 1,6-hexanediol diacrylate (B) or tetra(ethylene glycol) diacrylate (C) shown in Table 2 below, with a range of amine monomers as shown in Table 1 below a 1.1:1 ratio in Dichloro-methane (DCM) at a concentration of 5ml of DCM each 3.7 mmol of acrylate. In the above scheme, the letter 'R' is indicative of the particular organic group present in the monomers.
The polymerisation was then performed under stirring at 50 °C for 48 hours. PBAEs were precipitated through

pouring the reaction mixture in about 10 times the volume of diethyl-ether under vigorous mixing; the solvent was removed under vacuum.

The *Zeta potential of the PBAEs produced was measured.* Particle charge was determined using a Malvern Zetasizer Nano ZS (Malvern Instruments, Malvern, U.K.). Un-capped (acrylated terminated) PBAE were dissolved in 100 mM phosphate buffer (pH = 6.0) at about 20 mg/ml. Average electrophoretic motilities were measured at 25 °C and zeta potentials were calculated using the Smoluchowsky model.

All PBAEs exhibited positive charges, generally when no hydroxyl groups are present in the amine constituting the PBAE backbone, the charge is higher (about +15 mV) than when this group is present (about +8 mV).

## Example 2

### Preparation of end-capped PBAE derivatives

Acrylate-terminated polymers of Example 1 were dissolved in DCM at a concentration of 31.13% w/w. A range of end capping agents shown in Table 3 below were then added.

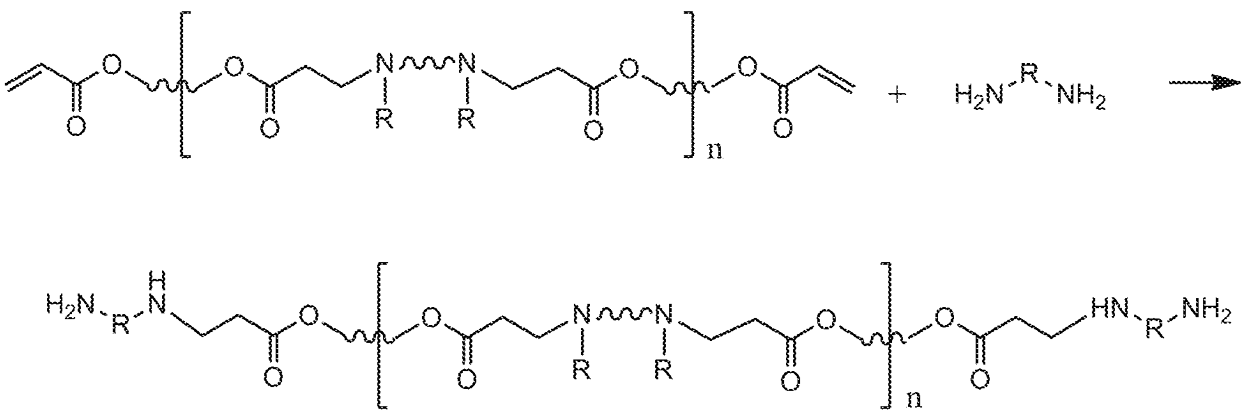

Specifically amine capping reagents (either ethylene diamine ('e1'), diethylenetriamine ('e2') or tris-(2-aminoethyl)amine ('e3') were dissolved in DCM to a concentration of 0.25 mol/l). The capping reactions were performed by mixing the polymer/DCM solution with amine solution at a ratio of 800 μl per 321 mg of polymer solution; the mixture was kept for 24 hours at room temperature.

End-capped PBAEs were recovered through precipitation in diethyl-ether under vigorous mixing, the unreacted amine were removed centrifuging the suspension of PBAE in diethyl-ether/DCM for 2 min at 1155 g. The supernatant was removed and the PBAEs washed twice with diethyl-ether. The end-capped PBAEs were then dried under vacuum.

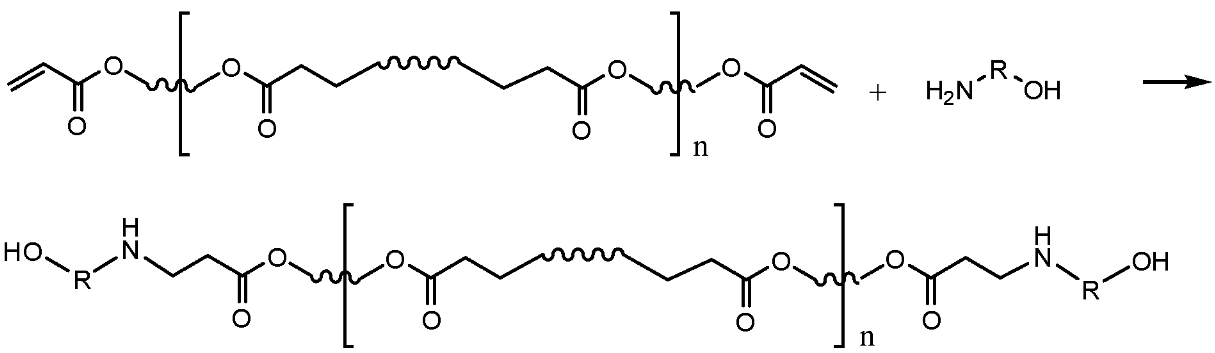

The method was repeated using a range of amino alcohols or instead of the diamines. The amino alcohols used were 2-aminethanol ('c2'), 3-amino-1-propanol ('c3'), 4-amino-1-butanol ('c4') and 5-amino-1-pentanol ('c5') to produce a range of compounds.

In addition, a mercapto alcohol (2-mercapto ethanol ('s2') was used instead of the amino alcohol in accordance with the following scheme.

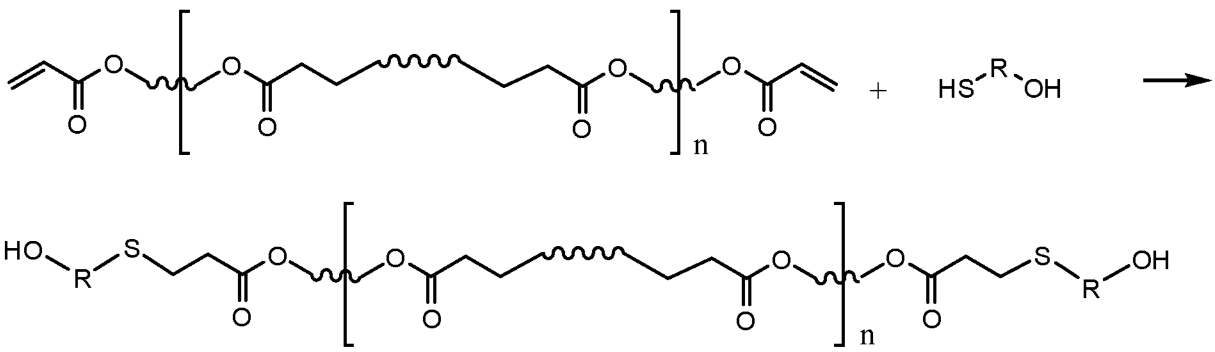

The PBAEs and derivatives obtained were coded through the constituents of the back bone using a capital letter to indicate the acrylate (A being 1,4-butanediol acrylate) and a number to indicate the amine as shown in Table 1; the further end capping is describes by a number preceded by the letter e. For example **A2-e1** is the PBAE obtained from 1,4-Butanediol diacrylate and 4,4'-Trimethylenedipiperidine then capped with ethylene-diamine.

Example 3

Preparation of Complex

(A) Preparation of Derivatised Agent

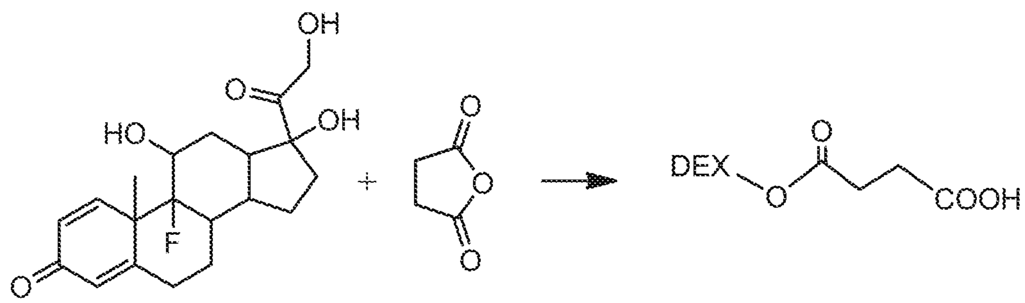

Dexamethasone (DEX) and 6α-Methyl-Prednisolone (Pred) were first succinylated by mixing 200 mg of DEX with 35 mg of succinic anhydride in 50 ml Dimethyl-formamide (DMF). The reaction was performed under nitrogen for 24 hours at room temperature with mixing. The solvent was removed under vacuum and the product (DEX-succ and Pred-succ) purified through dialysis.

In an alternative preparation, Dexamethasone (DEX) and 6a-Methyl-Prednisolone (Pred) were succinylated by mixing 200 mg of drug with 200 mg of succinic anhydride) and 10 mg of 4-(Dimethylamino)pyridine (DMAP) in 50 ml Dimethyl-formamide (DMF). The reaction was performed under nitrogen for 24 h at room temperature with mixing. The solvent was removed under vacuum and the solid residue (DEX-succ and Pred-succ) was purified through repeated washing with $dH_2O$. Finally, the product was dried under vacuum.

(B) Conjugation of Derivatised Agent to end capped PBAEs

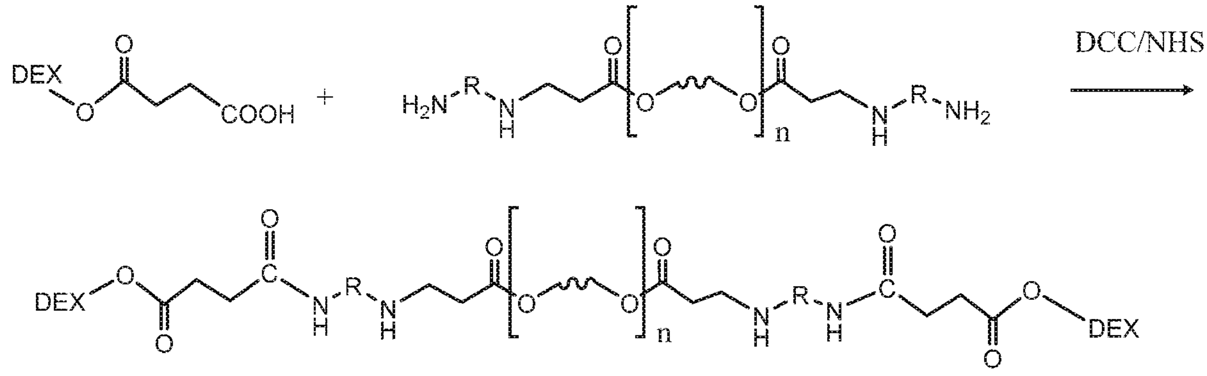


The conjugation of DEX-succ was performed by mixing 80 mg of end-capped PBAEs obtained in Example 2 with 8 mg of succinylated drug obtained in Example 3, 8 mg of 1-[3-(dimethylamino)-propyl]-3-ethylcarbodiimide hydrochloride (EDC) or 8 mg of N,N'-dicyclo-hexylcarbodiimide hydrochloride (DCC) and 8 mg of N-hydroxysulfosuccinimide (NHS) in 25 ml of DCM.

For Pred-succ conjugation 10 times the quantities employed for DEX-succ were used. After 24 hours at room temperature under mixing, the solution was filtered and the conjugates PBAEs-drug were precipitated through pouring the reaction mixture in about 10 times the volume of diethyl-ether under vigorous mixing; the solvent was removed under vacuum. PBAEs-drug conjugates were washed twice with diethylether. The final product was then dried under vacuum.

Example 4

Biological Data

A. Drug Penetration in cartilages

Bovine steers immature (7-day-old) feet were obtained from a local abattoir. Articular cartilage explants were surgically removed under sterile conditions from metacarpo-phalangeal joints. Full depth explants were excised using a 6mm diameter biopsy punch from the medial aspect of the medial condyle of individual joints. Explants were placed initially in Dulbecco's modified Eagles medium (DMEM; Invitrogen, Paisley, UK) and washed in the same medium to remove blood and small particulates due to the presence of a small amount of subchondral bone lining the basal aspect of cartilages.

GAG depleted samples were obtained digesting the samples in a solution of trypsin 1mg/ml in PBS for 24 hours at 37 °C, and these was also used in the tests described below.

A PTFE transport chamber was designed (Figure 2) and manufactured to study oneway diffusion of solutes entering into cartilage; the chamber walls were treated with casein to block non-specific binding of solutes to PTFE surfaces. Cartilage disks (6 mm diameter, 1 mm thick or ~0.4mm thick) were cut in half and placed in one of the holding slots machined into the chamber. The chamber facing the superficial zone was filled with 50 μl of a known concentration of PBAEs-drug solution supplemented with protease inhibitors; the other chamber side was filled with 50 μl of PBS containing protease inhibitors alone. The chamber was then placed in a petri dish containing d-$H_2O$ and covered to minimize evaporation, then placed inside an incubator at 37°C; stagnant layers at cartilage surfaces were prevented by placing the dish on a slow-speed rocker. At required intervals a sample was removed, washed in copious amount of water and placed in an Eppendorf containing 1 ml of digestion buffer.

Cartilages were digested using a sodium di-phosphate buffer 0.2 M containing 300 mg/l of papain, EDTA 1 mM and DTT 2mM. Samples were mixed in the buffer and incubated at 50 °C for 24 hours.

Experiments were performed on duplicate samples originated from 3 different animals.

Comparison of the drug penetration was performed against the results of a solution of the commercial drug formulation (for DEX, dexamethasone phosphate and for Pred, 6a-methyl-Prednisolone acetate) at the advised concentration of 4 mg/ml for DEX (equivalent to 4.4 mg/ml of dexamethasone phosphate) and 40 mg/ml for Pred (equivalent to 44 mg/ml 6a-methyl-Prednisolone acetate).

The amount of GAG present in the cartilages samples was determined through the DMMB (Dimethyl-Methylene Blue) assay as described in Farndale et al. (Biochim Biophys Acta. 1986;883(2): 173-177).

In addition, for DEX retention studies, cartilages were exposed to the DEX solution in the apparatus described above for 10 min and after washing in copious amount of water, they were place in an Eppendorf containing 0.5 ml of PBS. Cartilages were stored for up to 2.5 hours at 37 °C, at fixed intervals the cartilages were removed from the PBS solution, washed with water and placed in 1 ml of digestion buffer.

B. Agent quantification

Dexamethasone and 6α-methylprednisolone in the digestion buffer was quantified through reverse phase-HPLC. An Agilent series 1100 HPLC system was equipped with a TeknoKroma TRACE EXCEL 120 ODSB 5 μm analytical column thermostated at 25°C. The injection volume was 10 μl or 25 μl, the mobile phase was PBS:acetonitrile:glacial acetic acid 70:26:4 with a flow rate of 1 ml/min and the detector was a UV spectrophotometer at 244 nm. The amount of drug present in the cartilages was then expressed as mass of drug per cartilage mass.

**Results**

The results obtained were compared with those obtained using dexamethasone phosphate in the case of dexamethasone complexes and 6α-methylprednisolone acetate in the case of 6α-methylprednisolone complexes. Representative results are shown in Figure 4.

As is clear, the amount of DEX recovered from cartilages increased with time over the course of 10 min; the conjugation of DEX to PBAEs generally induced an increase with time of the ratio between the drug delivered through PBAEs against the commercial formulation of dexamethasone phosphate (DEX-P). The overall effect of PBAEs depended on the both the chain constituents and the end-capping agent (Figure 6).

The nature of the amine constituent of the polymer back-bone had a clear effect on the amounts obtained, the most effective being 4,4'-trimethylenedipiperidine, 3-(dimethylamino)-propylamine, 2-(2-pyridyl)ethylamine and 1-amino-2-propanol. These amines are not the most effective when PBAEs are used as non-viral DNA delivery system, highlighting the different requirements for drug delivery in cells against cartilages. Particularly, these amines present extra nitrogen capable of increasing the positive charge of the polymer chain, possibly increasing the electrostatic attraction towards the negatively charged biopolymers constituting the extracellular matrix of cartilages.

The investigation of the end-capping amine role in cartilages drug delivery showed that diethylentriamine was more effective than ethylene-diamine (Figure 6). This can be attributed to a steric effect as the longer chain of the end-capping amine could help the stability of the drug at the end of the chains, also the extra nitrogen present in diethylentriamine could results in higher a charge of the polymer conjugate.

When cartilages were exposed to the same concentration of DEX either as the commercial formulation of DEX-P or in the conjugated form to PBAE, the conjugation of Dexamethasone to PBAE resulted in a higher amount of the drug in the cartilages even after a very short period of time (1 min). The use of A2-e1-DEX resulted in the concentration of DEX in the cartilage to increase only in the first minute and then remain almost constant.

When the GAG depleted samples were used, it was found that despite relying on the negative charges of the GAG molecules to deliver the drug in the cartilages, PBAE were also effective on GAG depleted cartilage exhibiting about 40% of the untreated tissues.

DEX was quickly released from the cartilage; in normal GAG sample DEX concentration fell below detection limit after about 2 hours (Figure 11) in case of DEX-P; when PBAE-DEX were employed DEX was still detectable in the cartilage even after 2.5 hours). In GAG depleted cartilages the release was slower than in for normal GAG samples but the amount of DEX left in the tissue for PBAE-DEX was always higher than for DEX-P.

A more extensive investigation of the drug delivery properties of PBAE in cartilages revealed that many members of the PBAE family mildly enhanced the uptake of DEX, few candidates were not capable of increasing DEX concentration in cartilages, whilst some PBEA achieved remarkably high levels of DEX (Figure 6). Although no good coefficient of correlation (0.046) was found between zeta potential and diffusion coefficient of PBAE (Figure 8) or between zeta potential and DEX uptake (0.22) after 10 min exposure (Figure 7a); on the contrary good correlation was found between diffusion coefficient of DEX uptake (Figure 7b). Generally the compound e2 gave better performance than e1 or e3, the latter also generally did not perform as well as the other two.

End-capping with di-amine results in a $NH_2$ groups at the PBEA extremities and the formation of an amide bond with the succinilated DEX, however it is also possible to employ amino-alcohols or thio-alcohols compound to end-cap PBAEs. When these different compounds were used the conjugation of succinylated DEX returned an ester bond but the PBEA-DEX formed was still capable to increase DEX uptake (Figure 9) with efficacy dependent on the length of the amino-alcohol used. The optimal appeared to be a chain of about 3-4 carbons. Similar results were obtained for amino-ethanol and thio-alcohols.

PBAE were effective drug delivery system for cartilages also for another very commonly used steroidal drug such Prednisolone, also for this steroid the efficacy depended on the PBAE structure and end-capping; however the overall enhancement was greater for Prednisolone than for Dexamethasone (Figure 10).

By way of comparison, a solution containing 4 mg/ml of Dexamethsone and the same quantity of pure end-capped PBAE that would be found in PBAE-DEX was prepared and employed in similar cartilage uptake experiments to those described above. It was found however, that when pure PBAEs were present along with DEX, but the two compounds were not conjugated, the uptake of DEX was always lower than the corresponding case of pure steroidal

drug (Figure 12).

Example 5

Viability of chondrocytes after exposure to PBAE-DEX

The cartilage samples as described in Example 4 underwent a 1 hour predigestion stage with 0.4 % w/v pronase (Sigma, UK), dissolved in 4-(2-hydroxyethyl)-1-piperazineethanesulfonic acid (HEPES)-buffered DMEM (Gibco, UK) supplemented with 50 U/50 mg/mL penicillin-streptomycin (Sigma Aldrich) and 2.5 mg/mL amphotericin B. Cartilages samples were placed in sterile eppendorfs containing the filter-sterilized enzyme solution by passage through a 0.22-mm filter at a concentration of 1 ml solution for 5 cartilage extracts. The samples were incubated at 37 °C and the cartilage fragments were then washed twice with sterile PBS and digested with 1 ml each 6 cartilage sample of collagenase II (Sigma, UK) 0.1 % w/v for 16 h at 37 °C. Collagenase II was prepared in 4-(2-hydroxyethyl)-1-piperazineethanesulfonic acid (HEPES)-buffered DMEM (Gibco, UK) supplemented with 50 U/50 mg/mL penicillin-streptomycin (Sigma Aldrich and filter sterilised.

The triturate suspension was passed through a 100-mm nylon cell strainer (Fisher, UK) to remove matrix debris, and added to 10 mL DMEM/F-12 (1.1) with GlutaMAX (DMEM/F-12) (Gibco, UK) media, supplemented with 20% FBS, 1% penicillin-streptomycin and 1% amphotericin B. Chondrocytes were centrifuged at 1500 rpm at 4°C for 10 min to pellet the cells. The supernatant was discharged and the pelleted cells were washed in sterile PBS. After centrifugation at 1500 rpm at 4 °C for 10 min, the cells were suspended in supplemented 10 ml of DMEM/F-12 (10% FBS) and aseptically transferred in cell culture flasks. Chondrocytes were cultured in a humidified incubator at 37 °C and 5% $CO_2$. After 3 days chondrocytes were washed in sterile PBS and trypsinated. 24 well plates were the inoculated with approximately 6000 cells/well in 1 ml of DMEM/F-12 supplemented with 10% FBS and 1% pen-strep. Chondrocytes were grown for 2 days in a humidified incubator at 37 °C and 5% $CO_2$ before 15 μl of a solution containing DEX 4 mg/ml was added. The DEX solutions were prepared using Dexamethasone phosphate or PBAE-DEX conjugates.

Plates were incubated in a humidified incubator at 37 °C and 5% $CO_2$ for up to 3 days.

Chondrocytes viability was assessed through MTT and LDH assay kits (Sigma, UK) according to manufacturer's protocols. Experiments were performed in duplicate on cells originated from 3 different animals.

The results are shown in Figure 5. They showed that chondrocytes, which are cell present in the cartilages, did not suffer negatively from the exposure to PBAE for at least 3 days. Both enzyme assays (LDH and MTT) gave the same viability in samples exposed to DEX-P that samples exposed to PBAE-DEX.

Example 6

Determination of diffusion coefficient of PBAE in cartilages

In order to determine the basic diffusion coefficients of PBAEs in cartilages, Amino terminated PBAEs were fluoro-tagged (PBAE-FITC) using FluoroTag™ FITC Conjugation Kit (Sigma, UK) according to manufacturer's recommendations.

The diffusion coefficients of PBAE were determined using the same PTFE transport chamber and arrangements described above in Example 4. The chamber facing the superficial zone was filled with 50 μl of a known concentration of PBAEs-FITC supplemented with protease inhibitors; the other chamber side was filled with 50 μl of PBS containing protease inhibitors alone. After diffusion for a set length of time, 33μl of liquid for both chamber were removed and added to 67 μl of fresh PBS contained in black 96 well plate. Fluorescence was read using FLUOstar OPTIMA Microplate Reader (BMG Labtech, UK) with Ex = 480 nm and Em = 520 nm. The parameters were set assuring that the intensity response was in the linear range of concentrations as determined during PBAE-FITC purification.

The ratio of fluorescence between the two sides of the cartilage was plotted against diffusion time in order to identify the "break-through time" ($t_{lag}$), the diffusion coefficient ($\mathfrak{D}_{eff}$) was calculated as (Crank J. The mathematics of diffusion. Oxford, Clarendon Press; 1975):

$$\mathfrak{D}_{eff} = \frac{\delta^2}{6\, t_{lag}} \quad (1)$$

where:
*d* is the cartilage sample thickness

Experiments were performed on duplicate samples originated from 3 different animals.

Representative results for PBAEs A1-e1 and A2-e2 are shown in Figure 3. These show that PBAE can penetrate cartilages tissues and that the effective diffusion coefficient depends on the structure of the PBAE.

However, no good coefficient of correlation (0.046) was found between the zeta potential and diffusion coefficient of the PBAE (see Figure 7 hereinafter).

Example 8

Alternative Preparation of Complex

Complexes were formed using a method that did not require prior functionalization of the drug to be bound. The drugs in this case inherently presented a carboxyl group such in the case of (non-steroidal anti-inflammatory drugs (NSAIDs) like indomethacine and Ketorolac or in disease-modifying anti-rheumatic drugs (DMARDs) such as sulfasalazine and methotrexate. The bond between drug and PBAE can be formed directly. However in order to the drug to linked with an ester bond, PBAEs need to be end-capped with amino-alcohols or mercapto-alcohols instead of with di-amine as illustrated in the following schemes.

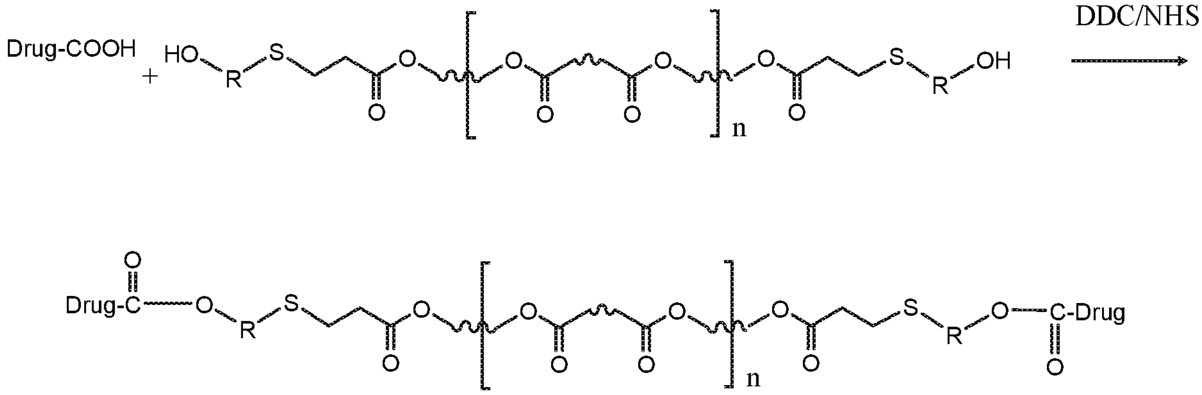


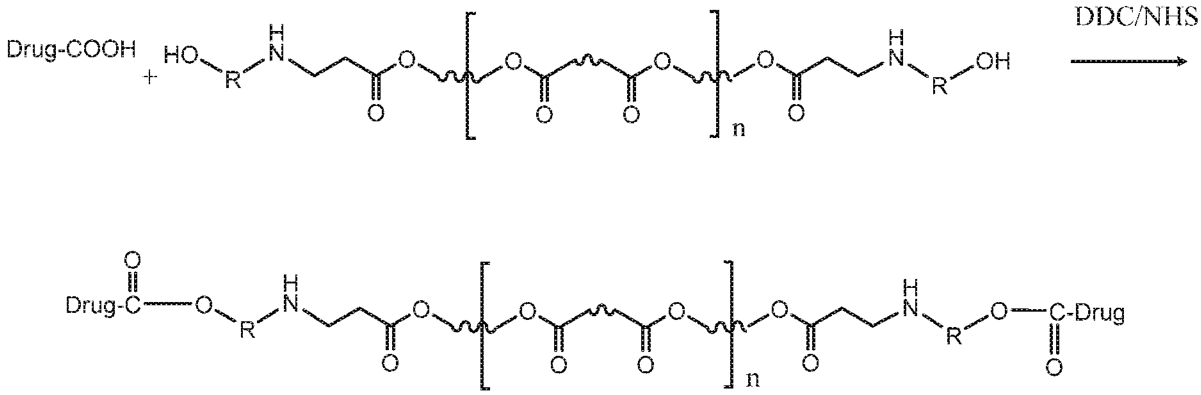


The conjugation of the NSAIDs and DMRAD was performed mixing 20 mg of end-capped PBAE with 2 mg of drug, 2 mg of N,N'-dicyclohexylcarbodiimide hydrochloride (DCC) and 2 mg of N-hydroxysulfosuccinimide (NHS) in 25 ml of DCM .

After 24 hours at room temperature under mixing, the solution was filtered and the conjugates PBAEs-drug were precipitated through pouring the reaction mixture in about 10 times the volume of diethyl-ether under vigorous mixing; the solvent was removed under vacuum. PBAEs-drug conjugates were washed twice with diethyl-ether:DCM (10:1). The final product was then dried under vacuum.

It was also possible to conjugate directly a drug exhibiting -OH (for example Dexamethasone or Prednisolone) to PBEA without succinylation. In this case, a heto-bifunctional linker (such as suberic acid) can be employed and this approach is irrespective of the end-capping step resulting in -OH or $-NH_2$ terminal groups as the ester bond is formed at one end of the linker in both cases, as illustrated in the following schemes.

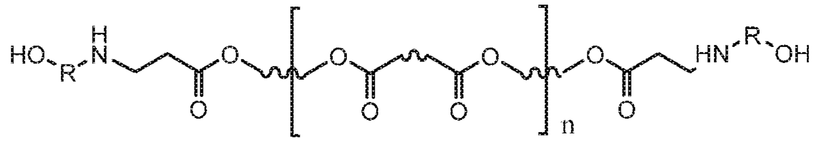

or

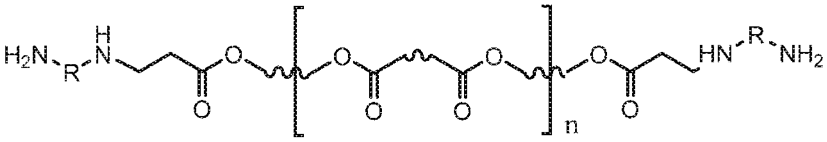

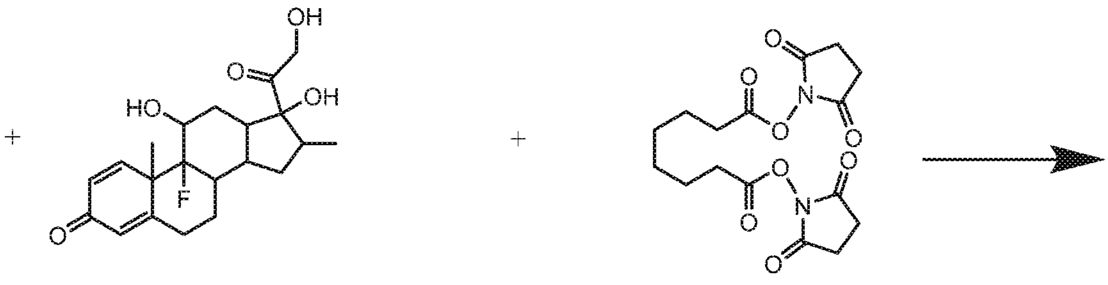

DEX

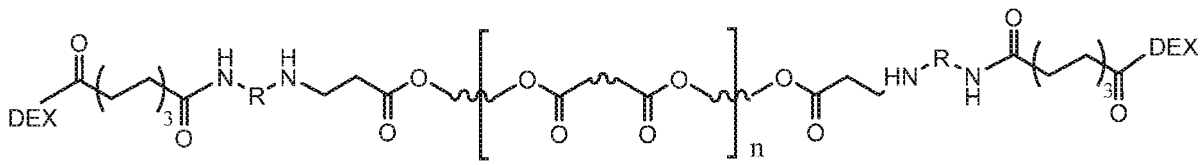

or

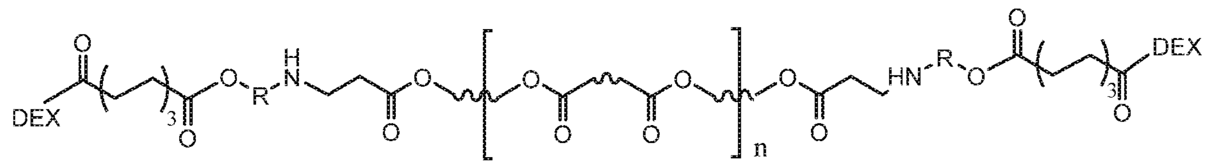

Dexmethasone, were conjugated to end-capped PBAE mixing 8 mg of drug with 80 mg of PBAE and 2 mg of Suberic acid bis(N-hydroxysuccinimide ester) in 25 ml of DCM; for 6α-methyl Prednisolone 10 times the mentioned quantities were used. After 24 hours at room temperature under mixing, the solution was filtered and the conjugates PBAEs-drug were precipitated through pouring the reaction mixture in about 10 times the volume of diethyl-ether under vigorous mixing; the solvent was removed under vacuum. PBAEs-drug conjugates were washed twice with diethyl-ether:DCM (10:1). The final product was then dried under vacuum.

Cartilage uptake experiments were carried out as described in Example 4 above. A Comparison of the drug penetration was performed against the results of a solution of the commercial drug formulation (for DEX, dexamethasone phosphate and for Pred, 6α-methyl-Prednisolone acetate) at the advised concentration of 4 mg/ml for DEX (equivalent to 4.4 mg/ml of dexamethasone phosphate) and 40 mg/ml for Pred (equivalent to 44 mg/ml 6α-methyl-Prednisolone acetate).

Ketorolac, Indomethacin and Sulfasalazine were quantified through reverse phase-HPLC. An Agilent series 1100 HPLC system was equipped with a TeknoKroma TRACE EXCEL 120 ODSB 5 μm analytical column thermostated at 25°C. The injection volume was 25 μl, the mobile phase with a flow rate of 1 ml/min was PBS:acetonitrile:glacial acetic acid 70:26:4 for Dexamethasone, Prednisolone, Ketorolac and Sulfasalazine whilst acetonitrile:acetate buffer pH=3.6 60:40 for Indomethacin. The detector was a UV spectrophotometer at 244 nm for Dexamethasone and Prednisolone, 255 nm for Indomethacin, 315 nm for Ketorolac and 360 nm for Sulfasalazine. The amount of drug present in the cartilages was then expressed as mass of drug per cartilage mass.

It was found that PBAE end-capped with amino alcohols or mercapto-alcohols could be employed to deliver non-steroidal anti-inflammatory drugs in cartilages. For Ketorolac A5 had greater efficacy than A12 regardless of the end-capping agent (Figure 13), the latter did not appear effective. Indomethacin and could only be delivered effectively

using A5 end-capped with short amino-alcohols (Figure 14). Disease modifying anti-rheumatoid drug (DMARD) such as sulfasalazine could also been delivery through the use of PBAE-drug (Figure 15). However, Dexamethasone and Prednisolone could also be delivered when conjugated in this manner (see Figures 16 and 17 respectively).

It is believed that the efficacy of PBAE as drug delivery systems for cartilages depends on the electrostatic attraction between polymer and cartilages constituents and therefore it was expected that the system would be relatively agnostic to the type of cargo to be delivered. This appears to be the case. Our results demonstrated that ketorolac was easier to deliver into cartilages than Indomethacin (the two examples of NSAID used), the reason could lie in the higher hydrophilicity of ketorolac than indomethacin, this was found to be the case also for the comparison between Prednisolone and Dexamethasone.

Sulfasalazine was employed as example of DMARD and the efficacy of PBAE was connected to the length of the end-capping agent.

The preparation of PBAE conjugated to steroids in a single step reaction is an important benefit as the reduction of reactions corresponds to lower production costs. The use of heto-bifunctional linker such as the NHS ester of subaric acid performs the same function of succinic anhydrade (forming an ester bond between steroid and linker) and forms another bond to the PBAE.

It appears therefore that the PBAE drug delivery system for cartilages is largely agnostic to the nature of the cargo. It may be used as an effective delivery system for steroids, NSAIDS and DMARDs. These may be bound to the PBAE directly if required, without prior functionalization.

## Example 9

### Alternative Complex Formation

It is possible to functionalise the drug in order for the molecule to exhbit either an amino group or a mercapto group, in this way the functionalised drug can be bind to the acrylate terminated PBAE during the end-capping reaction, for example as illustrated in Figure 1C.

In this case, a steroidal drug can be functionalised with amino acids forming an ester bond between the hydroxyl group on the drug and the carboxyl group of the aminoacid. Tthe amino group of the aminoacid on the functionalised steroidal drug can then bind directly to the acrylate groups present on the acrylate terminated PBAE as illustrated in the following scheme. In order to protect the amino group of aminoacid during the reaction with the steroids, a protecting group such as tert-Butoxy-carbonyl protected (BOC) aminoacids may be used. Similarly, thiolactones can be employed in order to for an ester bond between steroid and linker and forming a mercapto group that can bind to the acrylate group at the end of the PBAE.

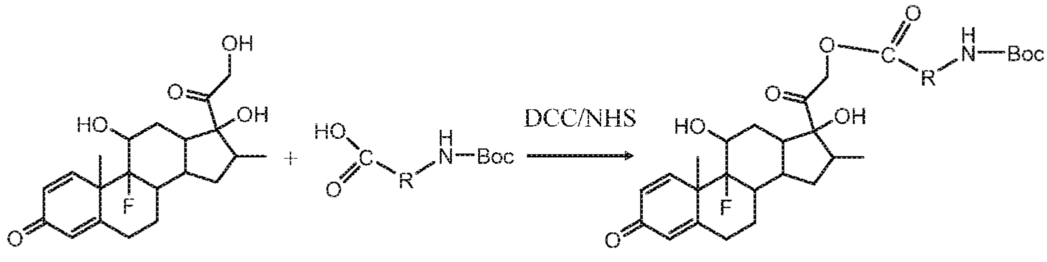


DEX

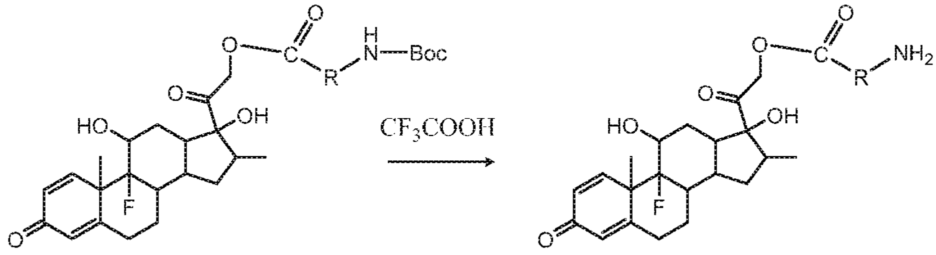


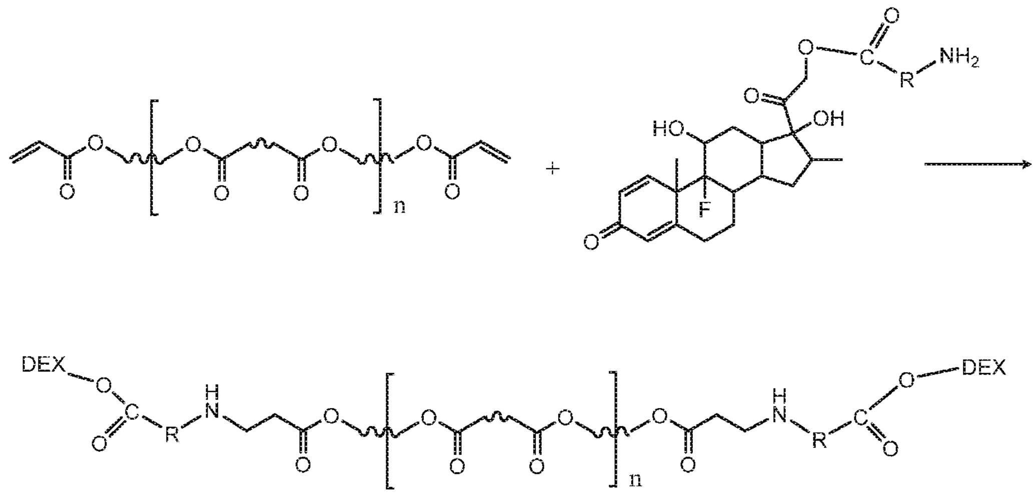

This method was effected by dissolving 50 mg of Dexamethasone (DEX) in 20 ml Dichloromethane (DCM) with 26 g of DCC and Dicyclohexylcarbodiimide and 6 mg of 4-(Dimethylamino)pyridine (DMAP). Then, 23 mg of N-(tert-Butoxycarbonyl)glycine (BOC-glycine) or 24.5 mg of BOC-β-alanine were added. The reaction was performed under nitrogen for 24 hours at room temperature with mixing. DCM was removed under reduced pressure to yield a white solid. The solid was re-dissolved in diethyl ether (10 ml) and washed twice with 10 ml 0.1N HCl. It was then dried over $MgSO_4$ and reduced under vacuum to yield white solid. The 21-O-t-Boc-glycinate ester or 21-O-t-Boc-β-alanine ester of Dexamethasone were purified by flash column chromatography.

The esters were dissolved in a 1:1 mixture of DCM and trifluoroacetic acid (TFA) and stirred at room Temperature; after 1 h, the reaction mixture was quenched with an aqueous saturated solution of $Na_2CO_3$ to neutrality. The mixture was extracted with DCM and the organic extracts were combined. Glycinate ester or β-alanine ester of Dexamethasone were obtained evaporating the solvent under reduced pressure.

Acrylate-terminated polymers (80 mg) and 10 mg of aminoacid ester of Dexamethasone were dissolved in 25 ml of DCM; the mixture was kept for 24 h at room temperature under mixing.

The PBAEs-DEX were recovered through precipitation in diethyl-ether under vigorous mixing, the unreacted aminoacid ester of DEX were removed centrifuging the suspension of PBAE in diethyl-ether/DCM for 2 min at 1155 g. The supernatant was removed and the PBAEs washed twice with diethyl-ether:DCM (10:1); PBAE-DEX were then dried under vacuum.

The resultant complexes were tested for cartilage uptake using the method of Example 4. The results are shown in Figure 18. Although preparation of PBAE-DEX through the functionalisation of DEX with aminoacids was possible, the drug delivery performance of the conjugate was not significantly better than the pure form of DEX, possibly due to the short chain length of the linker.

Example 10

Two step preparation of PBAE-drug Complex

PBAEs can be synthesised directly end-capped when the amine compound is in excess during the polymerisation, as illustrated in the following scheme.

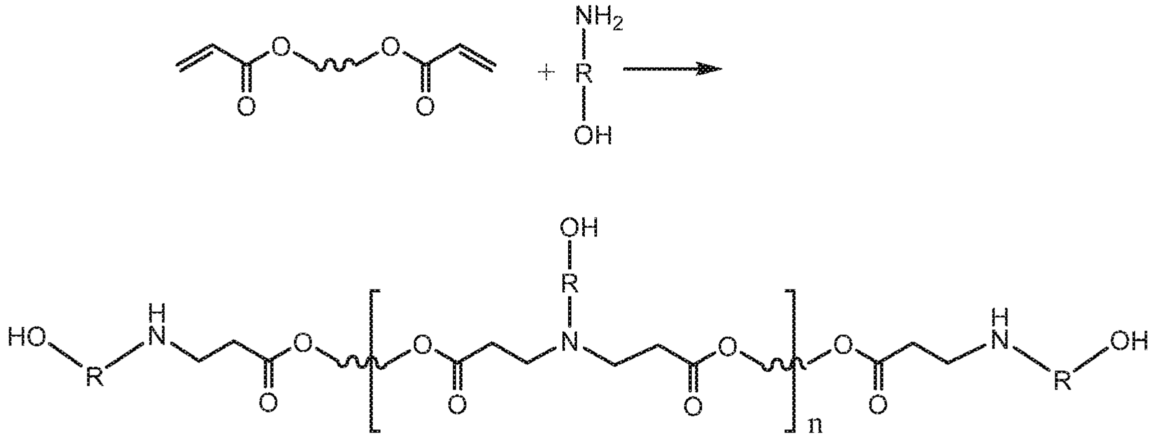

If such amine presents also another functional group that can be employed for conjugation, then a simpler two step approach to the preparation of PBAE-drug can be designed. This is possible for drugs exhibiting carboxyl groups (i.e Ketorolac, Indomethacoin, Sulfasalazine) or molecules exhibiting hydroxyl groups (i.e steroids) through the use of a heto-bifunctional linker as described in Example 8 above.

Hydroxyl terminated poly($\beta$-amino ester)s were synthesized by mixing diacrylate and amine monomers in a 1:1.1 ratio in Dichloro-methane (DCM) at a concentration of 5 ml of DCM (or THF) each 3.7 mmol of acrylate. The polymerisation was then performed under stirring at 50 °C for 48 hours. PBAEs were precipitated through pouring the reaction mixture in about 10 times the volume of diethyl-ether under vigorous mixing; the solvent was removed under vacuum.

The unreacted di-acrylate was removed dispersing the recovered polymer in diethyl-ether and centrifuging the suspension of PBAE for 2 min at 1155 g. The supernatant was removed and the PBAEs washed twice with diethyl-ether. The end-capped PBAEs were then dried under vacuum.

The conjugation of the Ketorolac was performed mixing 20 mg of end-capped PBAE with 2 mg of drug, 2 mg of N,N'-dicyclohexylcarbodiimide hydrochloride (DCC) and 2 mg of N-hydroxysulfosuccinimide (NHS) in 25 ml of DCM. Dexmethasone was conjugated to end-capped PBAE mixing 8 mg of drug with 80 mg of PBAE and 2 mg of Suberic acid bis(N-hydroxysuccinimide ester); for 6$\alpha$-methyl Prednisolone 10 times the mentioned quantities were used. After 24 hours at room Temperature under mixing, the solution was filtered and the conjugates PBAEs-drug were precipitated through pouring the reaction mixture in about 10 times the volume of diethyl-ether under vigorous mixing; the solvent was removed under vacuum. PBAEs-drug conjugates were washed twice with diethyl-ether:DCM (10:1). The final product was then dried under vacuum.

The cartilage uptake of the complexes was tested using the methods described in Example 4. The results are shown in Figure 19.

The amount of drug present in cartilages using PBAE-drug prepared in a two step synthesis was greater for the polymers prepared with acrylate B than acrylate A that has a with shorter carbon chain. The best improvement was seen for Prednisolone than for Dexamethasone; Ketorolac had the lowest improvement in drug uptake.

It appears therefore that when amino-alcohols are used in the PBAE backbone the polymer presents hydroxyl groups therefore the conjugation of drugs not necessary occurs to PBAE ends. The efficacy of PBAE-drug prepared in this way showed the already found patterned Pred>DEX>Ket.

It is possible to prepare PBAE-drug in a two steps synthesis protocol using amino-alcohols as polymer constituent in excess compared to acrylates and conjugating drugs with carboxyl groups directly or drugs with hydroxyl groups (i.e. steroids) through a heto-bifunctional linker.

Example 11

Conjugation of growth factors to PBAEs

A2-e1 and A5-e2, prepared as described in Example 2 above, were dissolved in (4-morphoiino)-ethane sulfonic acid (MES) buffer pH= 6.5 at a concentration of 1mg/ml. 1-[3-(dimethylamino)-propyl]-3-ethylcarbodiimide hydrochloride (EDC) and N-hydroxysulfosuccinimide sodium salt (sulfo-NHS) were dissolved in MES buffer at a concentration of 2 mg/l. TGF-β1 was dissolved in MES at a concentration 10 mg/l and another solution was prepared with FGF-2 at 100 mg/l. 50 μl of TGF-β1 solution were mixed with 10 μl of PBAE solution and 35 μl of the EDC/NHS sulfo solution. 50 μl of FGF-2 solution were mixed with 100 μl of PBAE solution and 350 μl of the EDC/NHS sulfo solution. Fresh MES was added to each growth factor solution to a final volume of 1.5 ml. The conjugation reaction was carried out at room temperature for 8 hours under mixing.

The PBAE-growth factors were purified with size exclusion chromatography (SEC) and lyophilised.

Example 12

Testing of Conjugates

Cartilages in vitro maturation

Cartilages samples were placed in a sterile well plate with a Dulbecco's Modified Eagles Medium (DMEM) immediately after removal from the joint, to remove blood and small particulates due to the presence of a small amount of subchondral bone lining the basal aspect of cartilages. Samples were incubate at 37°C in humidified atmosphere containing 5% $CO_2$.

After 24 hours the DMEM was removed and was replaced with serum-free culture medium with the following composition: DMEM high glucose, 4.4 g/ml; ascorbate-2-phospate, 100 μg/ml; penicillin 50 U/ml; streptomycin 50 μg/ml; HEPES pH 7.5, 10 μg/ml insulin, 10mM; Transferrin, 5.5 μg/ml and Selenium (ITS), 6.7 ng/ml. The media was also supplemented with growth factors: TGF-β1, 10 ng/ml and FGF-2, 100 ng/ml (Peprotech, London, UK) either pure or conjugated to PBAE; control samples were prepared without the presence of growth factors in the media. Cartilages were incubated at 37°C in humidified atmosphere containing 5% $CO_2$ and the media changed every 3 days.

Rheological determination of cartilages properties

Cartilage is a composite material, constituted by a solid portion (ECM) and a fluid portion (synovial fluid). Due to this feature, the cartilage responds to the onset of a stress with poro-elastic behaviour.

The evolution of the material properties of the cartilages during in vitro maturation was determined through rheological tests using AR-G2 (TA Instruments, UK) using 5 mm Peltier plates. Dynamic oscillation tests were performed; in these measurements a sinusoidal oscillation strain (σ) of small amplitude ($\sigma_0$) and frequency (ω):

$$\sigma(t) = \sigma_0 \exp(i\omega t) \quad (2)$$

was applied to the sample; the resulting stress (γ) was compared with the strain giving the complex modulus G*.

$$G^* = \frac{\sigma(t)}{\gamma(t)} \quad (3)$$

Because the two sinusoidal waves will have a phase angle difference, δ, the storage (G') and loss modulus (G") can be defined as the component in phase and π/2 out of phase with the strain, respectively.

$$G^* = G' + iG'' \quad (4)$$

and

$$G' = |G^*| \cos\delta \quad (5)$$

$$G'' = |G^*| \text{sen } \delta \tag{6}$$

Analysis was carried out at 37°C using frequency sweep between 0.1 and 100 Hz, a fixed strain of 0.1% and 25N applied load.

<u>Friction testing</u>

The frictional coefficient of cartilage explants was assessed using a pin-on-plate tribometer, with phosphate-buffered saline (PBS) as a lubricant. Six millimeter diameter freshly isolated and cultured articular cartilage explants, were fixed onto a nylon housing using cyanoacrylate. PBS was applied evenly over the polished glass surface, providing an average depth of approximately 1 mm. The tissue was then preloaded at 1.2 MPa for 120s prior to disc rotation to ensure consistent boundary lubrication as described previously (Neu et al. Arthritis Rheum 2010;62(9):2680e7).

The sliding speed was then ramped to 12 mm/s, before data was recorded for 15 seconds. Retrospective analysis to compute the mean frictional coefficient was then completed using MS Excel (Microsoft, Redmond, WA, USA) according to:

$$\mu = \frac{F}{N} \tag{7}$$

where:

F= frictional force;
$\mu$=frictional coefficient;
N=normal compressive force necessary to initiate sliding motion.

The value of F was determined through a previously obtained calibration of the rig.

**<u>Results</u>**

The mechanical properties of cartilages described by |G*| and δ revealed that without growth factors both parameters exhibited a slight increase during the first 3 days of in-vitro maturation and the gradually decreased (Figure 20) with the phase angles decreasing after 1 week. Instead, when growth factors (TGF-β1 and FGF-2) were added to the media, G* presented the greatest values after 1 week of maturation across the frequency range investigated (Figure 21).

The conjugation of the growth factor to PBAE resulted in the values of G* not decreasing after the maximum values reached after 1 week of maturation (Figures 22 and 23).

No significant difference was noticeable between conjugation to A5-e2 and A2-e1. The friction coefficient (Figure 25) during in vitro maturation increased when TGF-β1 and FGF-2 were added to the medium; moreover their absence resulted in the cartilages sample exhibiting the same friction coefficient than the immature samples (t = 0) for the incubation period (up to 3 weeks). When TGF-β1 and FGF-2 were conjugated to both A5-e2 and A1-e1 the progression of the friction coefficient during the in vitro maturation had the same patterned as the unconjugated growth factors but after 2 and 3 weeks the friction coefficient was higher.

In summary therefore, these results show that the cartilages mechanical properties evolve during in-vitro maturation, and the absence of any growth factors was detrimental as the G* quickly decreased through the frequency range tested and the friction coefficient did not increase; the addition of FGF-2 and TGF-β1 to the media had the beneficial effects expected and resulted in a maximum of mechanical properties after about 1 week, however this was followed by a decrease. Moreover, the conjugation of the growth factors visibly improved the properties of the cartilages as the maximum was reached after 3 days and no decrease was detected.

Thus, PBAE is an effective delivery system for cartilage not only for the transport of small drug molecules but also for biological molecules such as growth factors. Through PBAE-growth factors it is possible to increase in vitro cartilages maturation and maintain to developed mechanical properties for longer without degradation.

<u>Table 1.</u>

| | | |
|---|---|---|
| 1 | 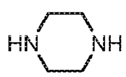 | piperazine |

(continued)

| | | |
|---|---|---|
| 2 | 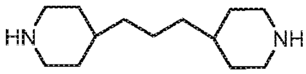 | 4,4'-Trimethylenedipiperidine |
| 3 | 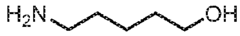 | 5-amino-1-pentanol |
| 4 | 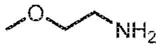 | 2-methoxyethylamine |
| 5 | 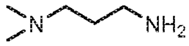 | 3-(dimethylamino)-1-propilamine |
| 6 | 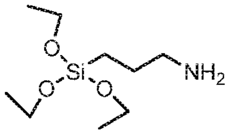 | (3-aminopropyl)triethoxysilane |
| 7 | 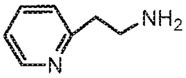 | 2-(2-pyridy)ethylamine |
| 8 | 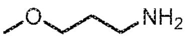 | 3-methoxy-propylamine |
| 9 | 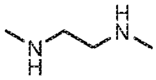 | N,N'-dimethylethyldiamine |
| 10 | 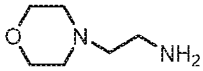 | 4-(2-aminoethyl)morpholine |
| 12 | 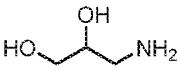 | 3-Amino-1,2-propanediol |
| 13 | 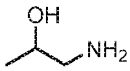 | DL 1-amino-2-propanol |
| 14 | 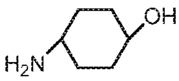 | trans-4-amino-hexanol |
| 15 | 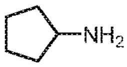 | Cyclopentylamine |
| 16 | 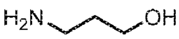 | 3-amino-1-propanol |

Table 2

| | | |
|---|---|---|
| A | 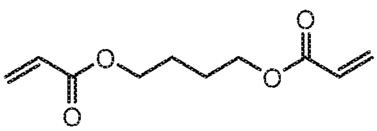 | 1,4-Butanediol diacrylate |
| B | 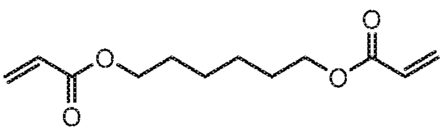 | 1,6 Hexanediol diacrylate |
| C | 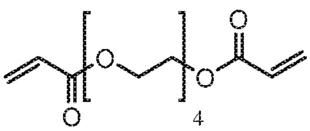 | Tetra(ethylene glycol) diacrylate |

Table 3

| | | |
|---|---|---|
| 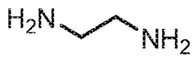 | Ethylen diamine | e1 |
| 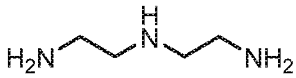 | Diethylentriamine | e2 |
| 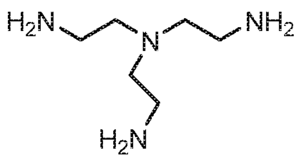 | Tris-(2-aminoethyl) amine | e3 |
| 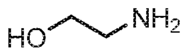 | 2-aminoethanol | c2 |
| 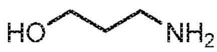 | 3 -amino-1-propanol | c3 |
| 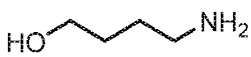 | 4-amino-1-butanol | c4 |
| 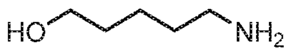 | 5-amino-1-pentanol | c5 |
| 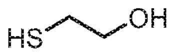 | 2-mercaptoethanol | s2 |

## Claims

1. A complex comprising a polymer comprising a poly(β-amino ester (PBAE) or a PBAE derivative having a functional group at an end of the polymer chain, and a therapeutic agent effective in the treatment of a cartilage disease or condition, for use in the treatment of cartilage damage, wherein the PBAE or PBAE derivative is covalently bound to the therapeutic agent and said complex is administered such that the complex penetrates pre-existing cartilage tissue.

2. The complex according to claim 1 for use in the treatment of cartilage damage wherein the PBAE is obtainable by reaction of a diacrylate monomer of formula (I)

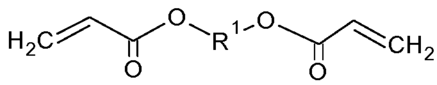

(I)

where $R^1$ is an optionally substituted divalent hydrocarbyl group, which may also be interposed with heteroatoms, with an amine monomer.

3. The complex according to claim 2 for use in the treatment of cartilage damage wherein the amine monomer is a compound of formula (II) or (III)

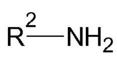

(II)

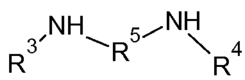

(III)

where $R^2$ is an optionally substituted hydrocarbyl group;
$R^3$ and $R^4$ are independently selected from optionally substituted hydrocarbyl groups;
$R^5$ is a divalent hydrocarbyl group which is optionally substituted and may also be interposed with heteroatoms such as oxygen;
or $R^3$ and/or $R^4$ are linked together or to $R^5$ to form one or more ring structures.

4. The complex according to claim 2 or claim 3 for use in the treatment of cartilage damage wherein, in the polymer, R1 is $C_{1-6}$alkylene group.

5. The complex according to claim 4 for use in the treatment of cartilage damage wherein $R^1$ is an n-butylene group.

6. The complex according to any one of claims 2 to 5 for use in the treatment of cartilage damage wherein the amine monomer is selected from 5-amino-1-pentanol, 2-methoxyethylamine, 3 -(dimethylamino)-propylamine, (3-aminopropyl) triethoxysilane, 2-(2-pyridyl)ethylamine, 3-methoxy-propylamine, 3-amino-1,2-propanediol, 1-amino-2 propanol, piperazine, 4,4-trimethylenedipiperidine, 3-(dimethylamino)-propylamine and N,N-dimethylethyldiamine.

7. The complex according to any one of claims 1 to 6 for use in the treatment of cartilage damage wherein the polymer is a PBAE derivative having amino or alcohol functional groups at the ends of the polymer chains forming 'end caps'.

8. The complex according to claim 7 for use in the treatment of cartilage damage wherein the PBAE derivative is of formula (VII) or (VIII)

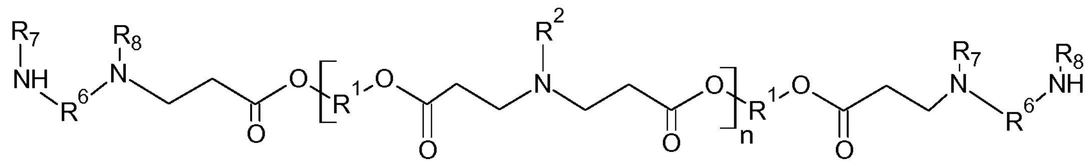

(VII)

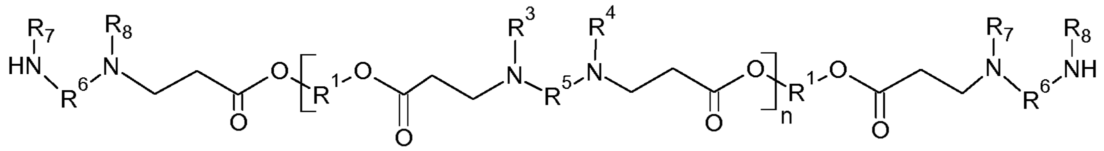

(VIII)

wherein $R^1$ is as defined in claim 2,
$R^2$ $R^3$, $R^4$ and $R^5$ are as defined in claim 3,
$R^6$ is an alkylene chain which is optionally interposed with a heteroatom,
$R^7$ and $R^8$ are the same and are selected from hydrogen or $C_{1-6}$alkyl,

and n is an integer of greater than 2.

9. The complex according to any one of claims 1 to 7 for use in the treatment of cartilage damage wherein the PBAE derivative is of formula (VIIA) or (VIIIA)

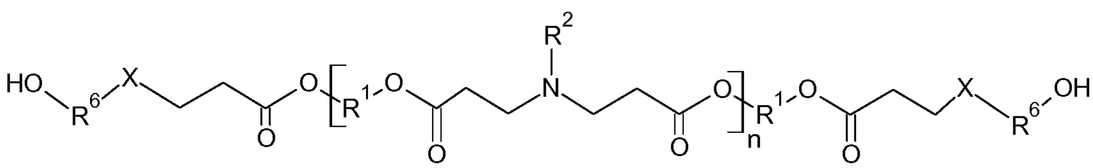

(VIIA)

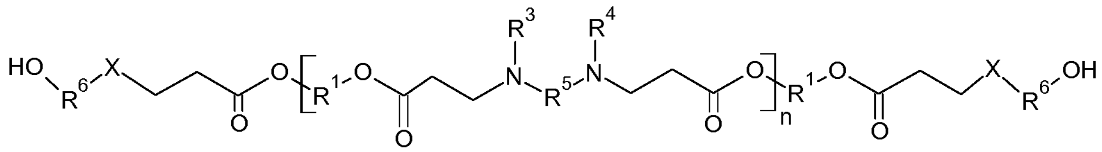

(VIIIA)

wherein $R^1$ is as defined in claim 2,
$R^2$ $R^3$, $R^4$ and $R^5$ are as defined in claim 3,
$R^6$ is an alkylene chain which is optionally interposed with a heteroatom, which may be substituted,
n is an integer of greater than 2, and X is NH or sulfur.

**10.** The complex according to claim 8 or claim 9 for use in the treatment of cartilage damage wherein $R^3$ and $R^4$ $C_{1-6}$alkyl; or $R^3$ and $R^4$ are linked together to form a piperazine ring; or $R^3$ and/or $R^4$ are linked to $R^5$ to form a piperidine ring.

**11.** The complex according to any one of claims 8 to 10 for use in the treatment of cartilage damage wherein $R^5$ is a $C_{1-10}$alkylene chain.

**12.** The complex according to any one of claims 8 to 11 for use in the treatment of cartilage damage wherein $R^7$ and $R^8$ are both hydrogen.

**13.** The complex according to claims 1-12 for use in the treatment of cartilage damage wherein the agent is a small drug molecule, biomacromolecule or a growth/maturation factor.

**14.** The complex according to claim 13 for use in the treatment of cartilage damage wherein the agent is a drug for use in the treatment of arthritis.

**15.** The complex according to claim 14 for use in the treatment of cartilage damage wherein the drug is a steroidal anti-inflammatory drug such as dexamethasone, prednisolone or 6$\alpha$-methylprednisolone; a DMARD such as methotrexate, sulfasalazine, a non-steroidal anti-inflammatory drug (NSAID) such as ibruprofen, naproxen, ketorolac tromethamine, Celecoxib and indomethacin or an AMPA receptor antagonist such as 2,3-dihydroxy-6-nitro-7-sulfamoyl-benzo[f]quinoxaline-2,3-dione (NBQX).

**16.** A pharmaceutical composition comprising a complex according to any one of claims 1 to 15 in combination with a pharmaceutically acceptable carrier for use in the treatment of cartilage damage.

**17.** A method for preparing a complex according to any one of the preceding claims, which method comprises reacting together a polymer which is a PBAE derivative as defined in to any one of claims 1 or 7 to 12 with an agent or a derivative thereof.

**18.** The method according to claim 17 wherein the agent is a succinylated derivative.

**19.** The method according to claim 17 or claim 18 wherein the PBAE derivative is obtained by reacting a PBAE with a compound of formula (VI)

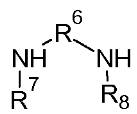

(VI)

where $R^6$ is an alkylene chain optionally interposed with an optionally substituted heteroatom, and $R^7$ and $R^8$ are the same and are selected from hydrogen or $C_{1-6}$alkyl.

**20.** The method according to claim 19 wherein the compound of formula (VI) is ethylene diamine, diethylenetriamine or tris-(2-aminoethyl) amine.

**21.** The method according to claim 17 or claim 18 wherein the PBAE derivative is obtained by reacting a PBAE compound

with a compound of formula (VIA)

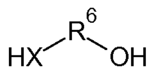

(VIA)

where $R^6$ is as defined above, and X is NH or sulfur.

**22.** The method according to claim 21 wherein the compound of formula (VIA) is 2-aminoethanol, 3-amino-1-propanol, 4-amino-1-butanol, 5-amino-1-pentanol or 2-mercaptoethanol.

**23.** The method according to any one of claims 19 to 21 wherein the PBAE used as a starting material is prepared by mixing together a monomer of formula (I) as defined in claim 2, with an appropriate amount of an amine monomer.

**24.** The method according to claim 23 wherein the amine monomer is a compound of formula (II) or (III) as defined in claim 3.

## Patentansprüche

**1.** Komplex, der ein Polymer, das einen Poly(β-aminoester (PBAE) oder ein PBAE-Derivat umfasst, das eine funktionelle Gruppe an einem Ende der Polymerkette aufweist, und ein Therapeutikum umfasst, das wirksam bei der Behandlung einer Knorpelkrankheit oder -erkrankung ist, zur Verwendung bei der Behandlung von Knorpelschäden, wobei der PBAE oder das PBAE-Derivat kovalent an das Therapeutikum gebunden ist und der Komplex derartig verabreicht wird, dass der Komplex bereits vorhandenes Knorpelgewebe durchdringt.

**2.** Komplex nach Anspruch 1 zur Verwendung bei der Behandlung von Knorpelschäden, wobei der PBAE durch Reaktion eines Diacrylat-Monomers der Formel (I) erhalten werden kann,

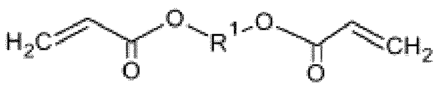

(I)

wobei $R^1$ für eine gegebenenfalls substituierte zweiwertige Hydrocarbylgruppe steht, in die außerdem Heteroatome, mit einem Amin-Monomer, eingefügt werden können.

**3.** Komplex nach Anspruch 2 zur Verwendung bei der Behandlung von Knorpelschäden, wobei das Amin-Monomer eine Verbindung der Formel (II) oder (III) ist,

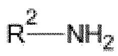

(II)

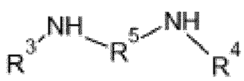

(III)

wobei $R^2$ eine gegebenenfalls substituierte Hydrocarbylgruppe ist;
$R^3$ und $R^4$ unabhängig aus gegebenenfalls substituierten Hydrocarbylgruppen ausgewählt sind;
$R^5$ für eine zweiwertige Hydrocarbylgruppe steht, die gegebenenfalls substituiert ist und in die möglicherweise ebenfalls Heteroatome wie etwa Sauerstoff eingefügt werden können;
oder $R^3$ und/oder $R^4$ miteinander oder mit $R^5$ verbunden sind, um eine oder mehrere Ringstrukturen zu bilden.

**4.** Komplex nach Anspruch 2 oder Anspruch 3 zur Verwendung bei der Behandlung von Knorpelschäden, wobei im Polymer R1 für eine $C_{1-6}$-Alkylengruppe steht.

**5.** Komplex nach Anspruch 4 zur Verwendung bei der Behandlung von Knorpelschäden, wobei $R^1$ für eine n-Butylengruppe steht.

6. Komplex nach einem der Ansprüche 2 bis 5 zur Verwendung bei der Behandlung von Knorpelschäden, wobei das Amin-Monomer aus Folgenden ausgewählt ist: 5-Amino-1-pentanol, 2-Methoxyethylamin, 3-(Dimethylamino)-propylamin, (3-Aminopropyl)-triethoxysilan, 2-(2-Pyridyl)ethylamin, 3-Methoxypropylamin, 3-Amino-1,2-propanediol, 1-Amino-2 propanol, Piperazin, 4,4-Trimethylenedipiperidin, 3-(Dimethylamino)-propylamin und N,N-Dimethylethyldiamin.

7. Komplex nach einem der Ansprüche 1 bis 6 zur Verwendung bei der Behandlung von Knorpelschäden, wobei das Polymer ein PBAE-Derivat ist, das funktionelle Amino- oder Alkoholgruppen an den Enden der Polymerketten aufweist, die "Endkappen" bilden.

8. Komplex nach Anspruch 7 zur Verwendung bei der Behandlung von Knorpelschäden, wobei das PBAE-Derivat die Formel (VII) oder (VIII) hat

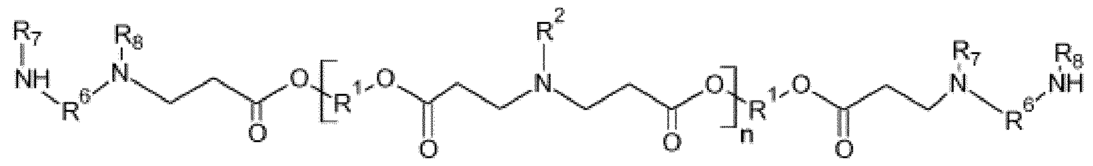

(VII)

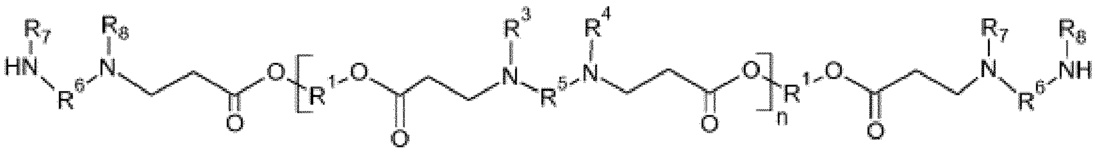

(VIII)

wobei $R^1$ wie in Anspruch 2 definiert ist,
$R^2$, $R^3$, $R^4$ und $R^5$ wie in Anspruch 3 definiert sind,
$R^6$ eine Alkylenkette ist, in die gegebenenfalls ein Heteroatom eingefügt ist,
$R^7$ und $R^8$ gleich sind und aus Wasserstoff oder $C_{1-6}$-Alkyl ausgewählt sind,

und n für eine ganze Zahl größer als 2 steht.

9. Komplex nach einem der Ansprüche 1 bis 7 zur Verwendung bei der Behandlung von Knorpelschäden, wobei das PBAE-Derivat die Formel (VIIA) oder (VIIIA) hat

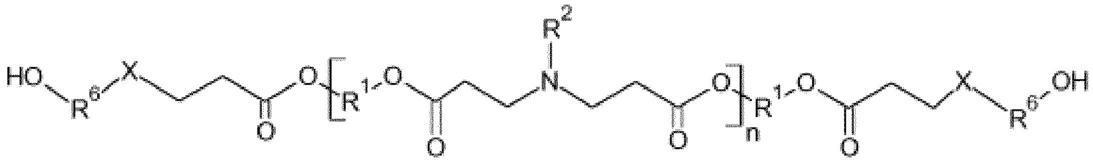

(VIIA)

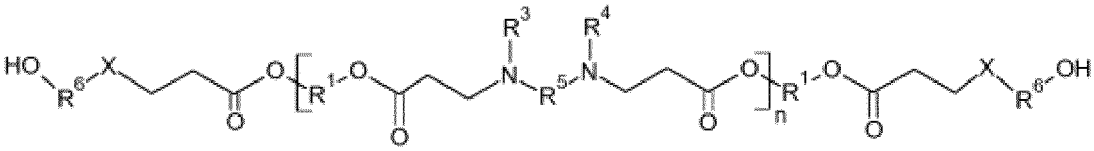

(VIIIA)

wobei $R^1$ wie in Anspruch 2 definiert ist,

$R^2$, $R^3$, $R^4$ und $R^5$ wie in Anspruch 3 definiert sind,
$R^6$ eine Alkylenkette ist, in die gegebenenfalls ein Heteroatom eingefügt ist, das substituiert werden kann,
n für eine ganze Zahl größer als 2 steht, und X für NH oder Schwefel steht.

**10.** Komplex nach Anspruch 8 oder Anspruch 9 zur Verwendung bei der Behandlung von Knorpelschäden, wobei $R^3$ und $R^4$ $C_{1-6}$alkyl; oder $R^3$ and $R^4$ miteinander verbunden sind, um einen Piperazinring zu bilden; oder $R^3$ und/oder $R^4$ mit $R^5$ verbunden sind, um einen Piperidinring zu bilden.

**11.** Komplex nach einem der Ansprüche 8 bis 10 zur Verwendung bei der Behandlung von Knorpelschäden wobei $R^5$ für eine $C_{1-10}$-Alkylenkette steht.

**12.** Komplex nach einem der Ansprüche 8 bis 11 zur Verwendung bei der Behandlung von Knorpelschäden wobei sowohl $R^7$ als auch $R^8$ für Wasserstoff stehen.

**13.** Komplex nach den Ansprüchen 1-12 zur Verwendung bei der Behandlung von Knorpelschäden, wobei der Wirkstoff ein kleines Arzneimittelmolekül, ein Biomakromolekül oder ein Wachstums-/Reifungsfaktor ist.

**14.** Komplex nach Anspruch 13 zur Verwendung bei der Behandlung von Knorpelschäden, wobei der Wirkstoff ein Arzneimittel zur Verwendung bei der Behandlung von Arthritis ist.

**15.** Komplex nach Anspruch 14 zur Verwendung bei der Behandlung von Knorpelschäden, wobei das Arzneimittel ein steroidales Antiphlogistikum, wie etwa Dexamethason, Prednisolon oder 6$\alpha$-Methylprednisolon; ein DMARD, wie etwa Methotrexat, Sulfasalazin, ein nichtsteroidales Antiphlogistikum (non-steroidal anti-inflammatory drug - NSAID), wie etwa Ibuprofen, Naproxen, Ketorolac-tromethamin, Celecoxib und Indomethacin, oder ein AMPA-Rezeptorantagonist, wie etwa 2,3-Dihydroxy-6-nitro-7-sulfamoyl-benzo[f]quinoxalin-2,3-dion (NBQX) ist.

**16.** Pharmazeutische Zusammensetzung, die einen Komplex nach einem der Ansprüche 1 bis 15 in Kombination mit einem pharmazeutisch unbedenklichen Träger zur Verwendung bei der Behandlung von Knorpelschäden umfasst.

**17.** Verfahren zur Herstellung eines Komplexes nach einem der vorhergehenden Ansprüche, wobei das Verfahren das Zurreaktionbringen eines Polymers, das ein PBAE-Derivat wie in einem der Ansprüche 1 oder 7 bis 12 definiert ist, mit einem Wirkstoff oder einem Derivat davon umfasst.

**18.** Verfahren nach Anspruch 17, wobei der Wirkstoff ein succinyliertes Derivat ist.

**19.** Verfahren nach Anspruch 17 oder Anspruch 18, wobei das PBAE-Derivat erlangt wird durch das Reagieren eines PBAE mit einer Verbindung der Formel (VI)

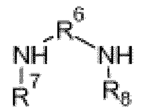

(VI)

wobei $R^6$ für eine Alkylenkette steht, in die gegebenenfalls ein gegebenenfalls substituiertes Heteroatom eingefügt ist, und $R^7$ und $R^8$ gleich und aus Wasserstoff oder $C_{1-6}$-Alkyl ausgewählt sind.

**20.** Verfahren nach Anspruch 19, wobei die Verbindung der Formel (VI) Ethylendiamin, Diethylentriamin oder Tris-(2-aminoethyl)-amin ist.

**21.** Verfahren nach Anspruch 17 oder Anspruch 18, wobei das PBAE-Derivat erlangt wird durch das Reagieren einer PBAE-Verbindung mit einer Verbindung der Formel (VIA)

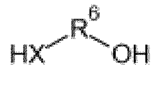

(VIA)

wobei R6 für das gleiche wie vorstehend definiert steht, und X für NH oder Schwefel steht.

**22.** Verfahren nach Anspruch 21, wobei die Verbindung der Formel (VIA) 2-Aminoethanol, 3-Amino-1-propanol, 4-Amino-1-butanol, 5 -Amino-1-pentanol oder 2-Mercaptoethanol ist.

**23.** Verfahren nach einem der Ansprüche 19 bis 21, wobei der PBAE, der als ein Ausgangsmaterial verwendet wird, durch das Zusammenmischen eines Monomers der Formel (I), wie in Anspruch 2 definiert, mit einer angemessenen Menge an Amin-Monomer hergestellt wird.

**24.** Verfahren nach Anspruch 23, wobei das Amin-Monomer eine Verbindung der Formel (II) oder (III) nach Anspruch 3 ist.

## Revendications

**1.** Complexe comprenant un polymère comprenant un poly(β-amino ester) (PBAE) ou un dérivé de PBAE ayant un groupe fonctionnel à une extrémité de la chaîne polymère, et un agent thérapeutique efficace dans le traitement d'une maladie ou affection d'un cartilage, pour une utilisation dans le traitement d'une lésion d'un cartilage, dans lequel le PBAE ou le dérivé de PBAE est lié par liaison covalente à l'agent thérapeutique et ledit complexe est administré de façon que le complexe pénètre dans un tissu cartilagineux préexistant.

**2.** Complexe selon la revendication 1 pour une utilisation dans le traitement d'une lésion d'un cartilage dans lequel PBAE peut être obtenu par la réaction d'un monomère de diacrylate de formule (I)

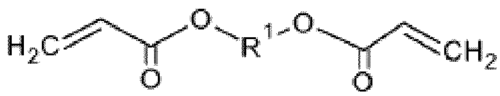

(I)

où $R^1$ est un groupe hydrocarbyle divalent facultativement substitué, qui peut également être intercalé avec des hétéroatomes, avec un monomère d'amine.

**3.** Complexe selon la revendication 2 pour une utilisation dans le traitement d'une lésion d'un cartilage dans lequel le monomère d'amine est un composé de formule (II) ou (III)

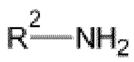

(II)

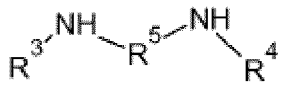

(III)

où $R^2$ est un groupe hydrocarbyle facultativement substitué ;
$R^3$ et $R^4$ sont sélectionnés indépendamment parmi des groupes hydrocarbyles facultativement substitués ;
$R^5$ est un groupe hydrocarbyle divalent qui est facultativement substitué et qui peut également être intercalé avec des hétéroatomes tels qu'un oxygène ;
ou $R^3$ et/ou $R^4$ sont liés ensemble ou à $R^5$ pour former une ou plusieurs structures cycliques.

**4.** Complexe selon la revendication 2 ou la revendication 3 pour une utilisation dans le traitement d'une lésion d'un cartilage dans lequel, dans le polymère, R1 est un groupe alkylène en $C_1$ à $C_6$.

**5.** Complexe selon la revendication 4 pour une utilisation dans le traitement d'une lésion d'un cartilage dans lequel $R^1$ est un groupe n-butylène.

**6.** Complexe selon l'une quelconque des revendications 2 à 5 pour une utilisation dans le traitement d'une lésion d'un cartilage dans lequel le monomère d'amine est sélectionné parmi le 5-amino-1-pentanol, la 2-méthoxy-éthylamine, la 3-(diméthylamino)-propylamine, le (3-amino-propyl)triéthoxysilane, la 2-(2-pyridyl)éthylamine, la 3-méthoxy-propylamine, le 3-amino-1,2-propanediol, le 1-amino-2-propanol, la pipérazine, la 4,4-triméthylène-dipipéridine, la 3-(diméthylamino)-propylamine et la N,N-diméthyléthylamine.

**7.** Complexe selon l'une quelconque des revendications 1 à 6 pour une utilisation dans le traitement d'une lésion d'un cartilage dans lequel le polymère est un dérivé de PBAE ayant des groupes fonctionnels amino ou alcool aux extrémités des chaînes polymères formant des « coiffes terminales ».

**8.** Complexe selon la revendication 7 pour une utilisation dans le traitement d'une lésion d'un cartilage dans lequel le dérivé de PBAE est de formule (VII) ou (VIII)

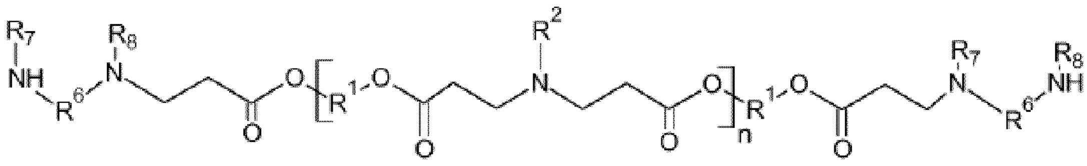

(VII)

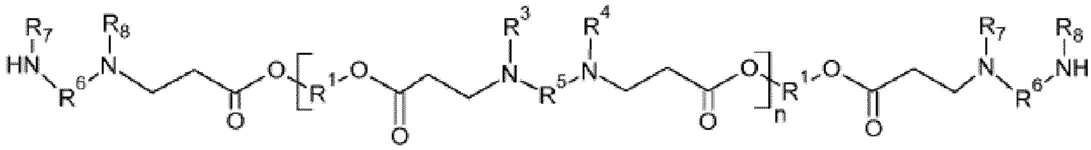

(VIII)

dans lesquelles $R^1$ est tel que défini dans la revendication 2, $R^2$, $R^3$, $R^4$ et $R^5$ sont tels que définis dans la revendication 3, $R^6$ est une chaîne alkylène qui est facultativement intercalée avec un hétéroatome,
$R^7$ et $R^8$ sont identiques et sont sélectionnés parmi un hydrogène ou un alkyle en $C_1$ à $C_6$,
et n est un nombre entier supérieur à 2.

**9.** Complexe selon l'une quelconque des revendications 1 à 7 pour une utilisation dans le traitement d'une lésion d'un cartilage dans lequel le dérivé de PBAE est de formule (VIIA) ou (VIIIA)

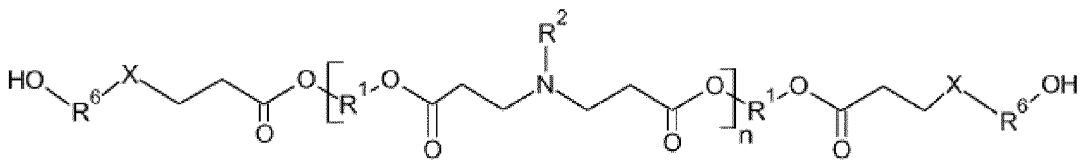

(VIIA)

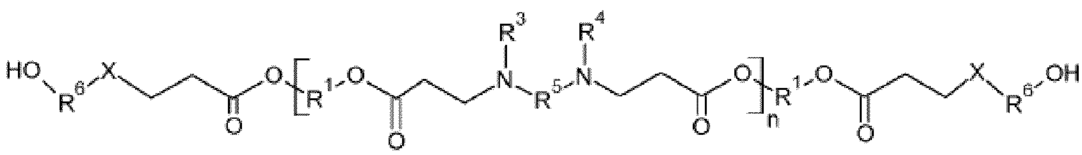

(VIIIA)

dans lesquelles $R^1$ est tel que défini dans la revendication 2, $R^2$, $R^3$, $R^4$ et $R^5$ sont tels que définis dans la revendication 3, $R^6$ est une chaîne alkylène qui est facultativement intercalée avec un hétéroatome, qui peut être substitué,
n est un nombre entier supérieur à 2, et X est NH ou un soufre.

**10.** Complexe selon la revendication 8 ou la revendication 9 pour une utilisation dans le traitement d'une lésion d'un cartilage dans lequel $R^3$ et $R^4$ sont un alkyle en $C_1$ à $C_6$ ; ou $R^3$ et $R^4$ sont liés ensemble pour former un cycle pipérazine ; ou $R^3$ et/ou $R^4$ sont liés à $R^5$ pour former un cycle pipéridine.

**11.** Complexe selon l'une quelconque des revendications 8 à 10 pour une utilisation dans le traitement d'une lésion d'un cartilage dans lequel $R^5$ est une chaîne alkylène en $C_1$ à $C_{10}$.

**12.** Complexe selon l'une quelconque des revendications 8 à 11 pour une utilisation dans le traitement d'une lésion d'un cartilage dans lequel $R^7$ et $R^8$ sont tous les deux un hydrogène.

**13.** Complexe selon les revendications 1 à 12 pour une utilisation dans le traitement d'une lésion d'un cartilage dans lequel l'agent est une petite molécule de médicament, une biomacromolécule ou un facteur de croissance/maturation.

**14.** Complexe selon la revendication 13 pour une utilisation dans le traitement d'une lésion d'un cartilage dans lequel l'agent est un médicament pour une utilisation dans le traitement d'une arthrite.

**15.** Complexe selon la revendication 14 pour une utilisation dans le traitement d'une lésion d'un cartilage dans lequel le médicament est un médicament anti-inflammatoire stéroïdien tel que la dexaméthasone, la prednisolone ou la 6α-méthylprednisolone ; un ARMM tel que le méthotrexate, la sulfasalazine, un médicament anti-inflammatoire non stéroïdien (AINS) tel que l'ibuprofène, le naproxène, le kétorolac trométhamine, le célécoxib et l'indométhacine ou un antagoniste des récepteurs AMPA tels que la 2,3-dihydroxy-6-nitro-7-sulfamoyl-benzo[f] quinoxaline-2,3-dione (NBQX).

**16.** Composition pharmaceutique comprenant un complexe selon l'une quelconque des revendications 1 à 15 en combinaison avec un support pharmaceutiquement acceptable pour une utilisation dans le traitement d'une lésion d'un cartilage.

**17.** Procédé pour préparer un complexe selon l'une quelconque des revendications précédentes, lequel procédé comprend la réaction ensemble d'un polymère qui est un dérivé de PBAE tel que défini dans l'une quelconque des revendications 1 ou 7 à 12 avec un agent ou un dérivé de celui-ci.

**18.** Procédé selon la revendication 17 dans lequel l'agent est un dérivé succinylé.

**19.** Procédé selon la revendication 17 ou la revendication 18 dans lequel le dérivé de PBAE est obtenu par la réaction d'un PBAE avec un composé de formule (VI)

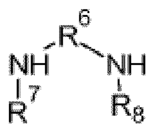

(VI)

où $R^6$ est une chaîne alkylène facultativement intercalée avec un hétéroatome facultativement substitué, et $R^7$ et $R^8$ sont identiques et sont sélectionnés parmi un hydrogène ou un alkyle en $C_1$ à $C_6$.

**20.** Procédé selon la revendication 19 dans lequel le composé de formule (VI) est l'éthylène diamine, la diéthylène triamine ou la tris-(2-aminoéthyl)amine.

**21.** Procédé selon la revendication 17 ou la revendication 18 dans lequel le dérivé de PBAE est obtenu par la réaction d'un composé de PBAE avec un composé de formule (VIA)

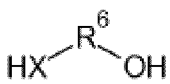

(VIA)

où $R^6$ est tel que défini ci-dessus, et X est NH ou un soufre.

**22.** Procédé selon la revendication 21 dans lequel le composé de formule (VIA) est le 2-aminoéthanol, le 3-amino-1-propanol, le 4-amino-1-butanol, le 5-amino-1-pentanol ou le 2-mercaptoéthanol.

**23.** Procédé selon l'une quelconque des revendications 19 à 21 dans lequel le PBAE utilisé comme composé de départ

est préparé en mélangeant ensemble un monomère de formule (I) tel que défini dans la revendication 2, avec une quantité appropriée d'un monomère d'amine.

**24.** Procédé selon la revendication 23 dans lequel le monomère d'amine est un composé de formule (II) ou (III) telle que définie dans la revendication 3.

Figure 1

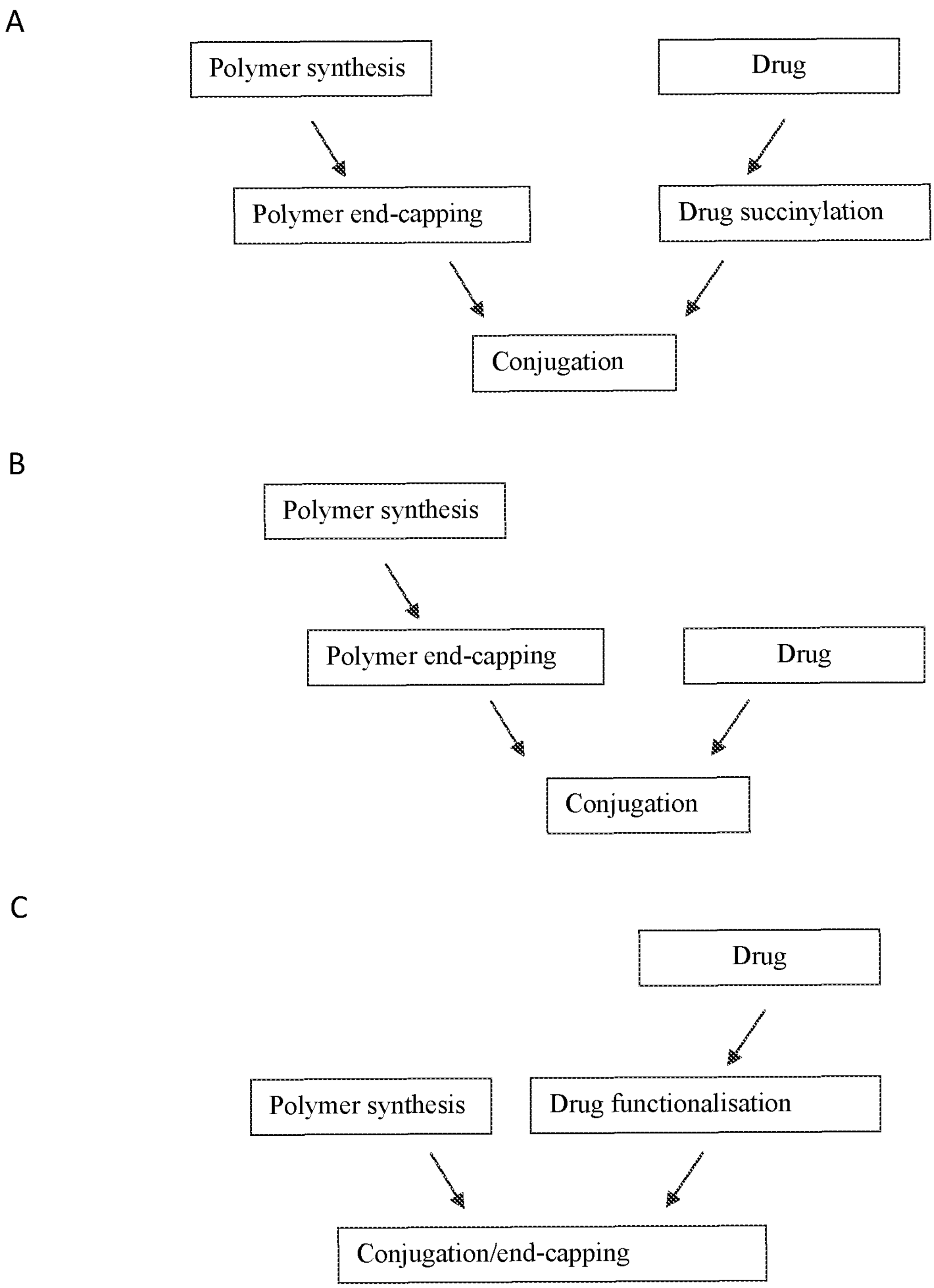
A
Polymer synthesis
Drug
Polymer end-capping
Drug succinylation
Conjugation
B
Polymer synthesis
Polymer end-capping
Drug
Conjugation
C
Drug
Polymer synthesis
Drug functionalisation
Conjugation/end-capping

D

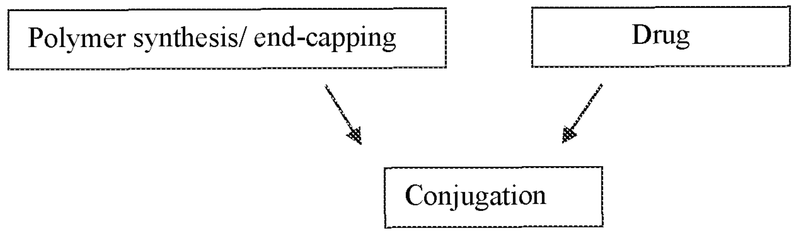


Figure 2

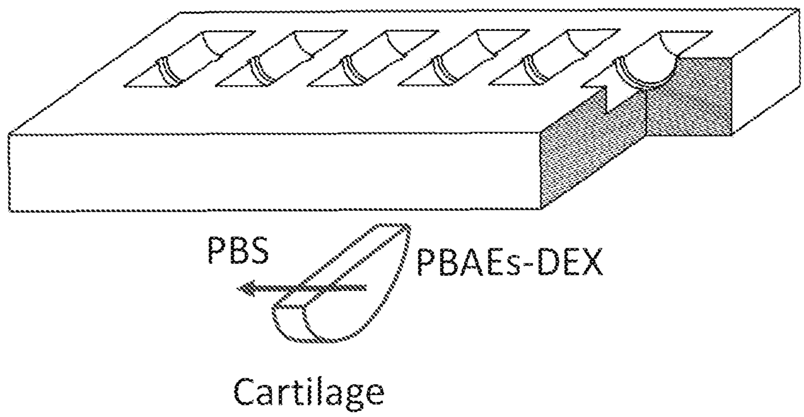


Figure 3

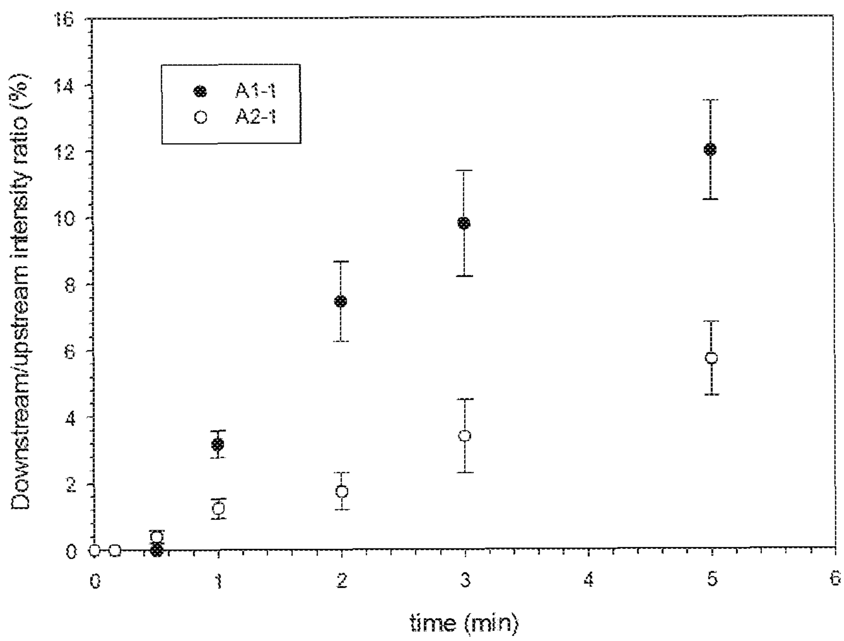

Figure 4

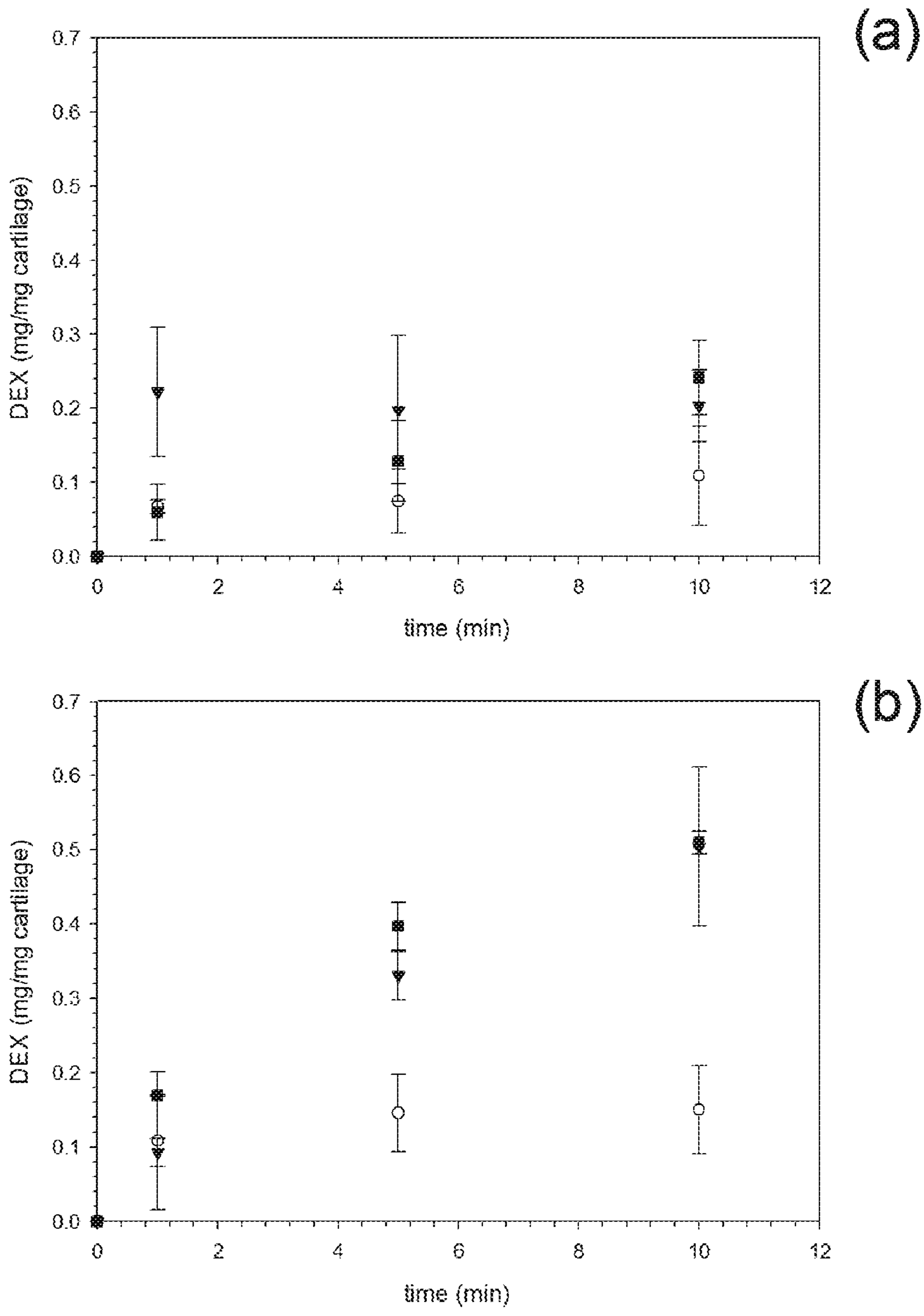
(a)
0.7
0.6
0.5
0.4
0.3
0.2
0.1
0.0
DEX (mg/mg cartilage)
0
2
4
6
8
10
12
time (min)
(b)
0.7
0.6
0.5
0.4
0.3
0.2
0.1
0.0
DEX (mg/mg cartilage)
0
2
4
6
8
10
12
time (min)

Figure 5

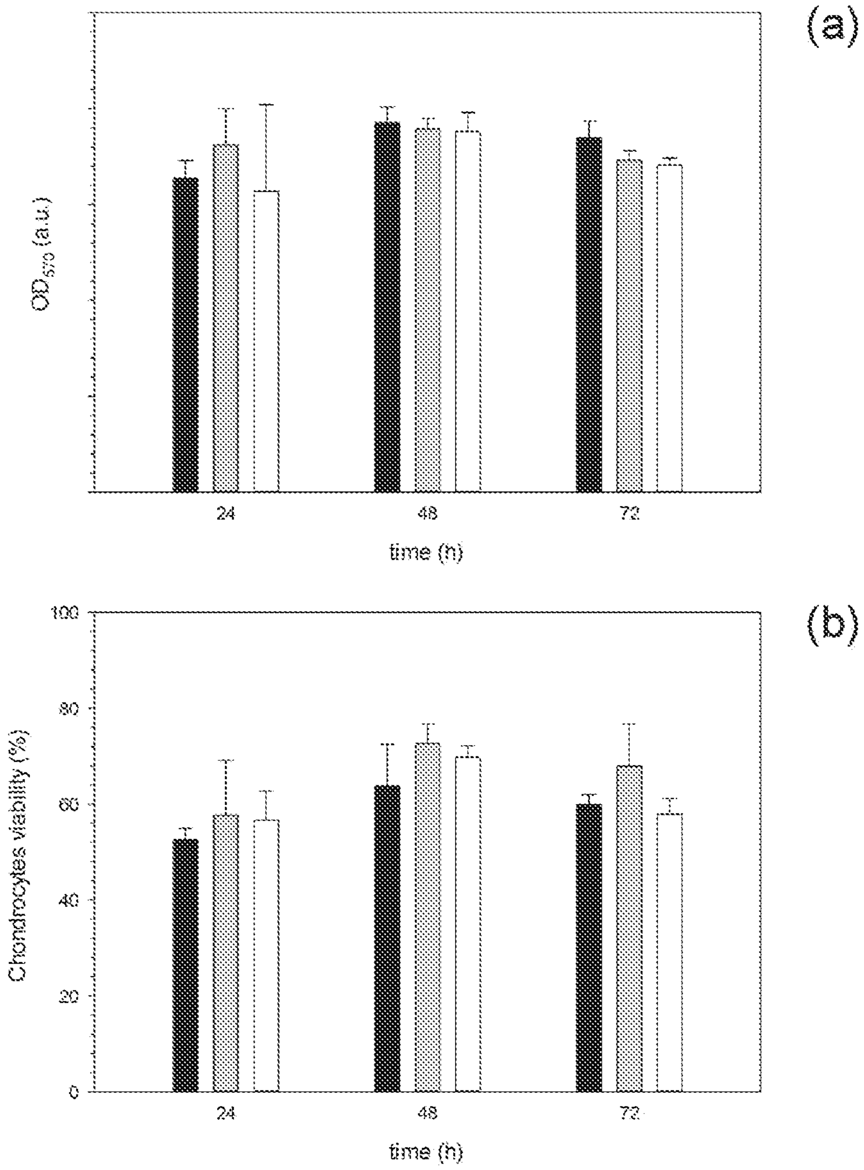
(a)
$OD_{570}$ (a.u.)
24
48
72
time (h)
(b)
Chondrocytes viability (%)
0
20
40
60
80
100
24
48
72
time (h)

Figure 6

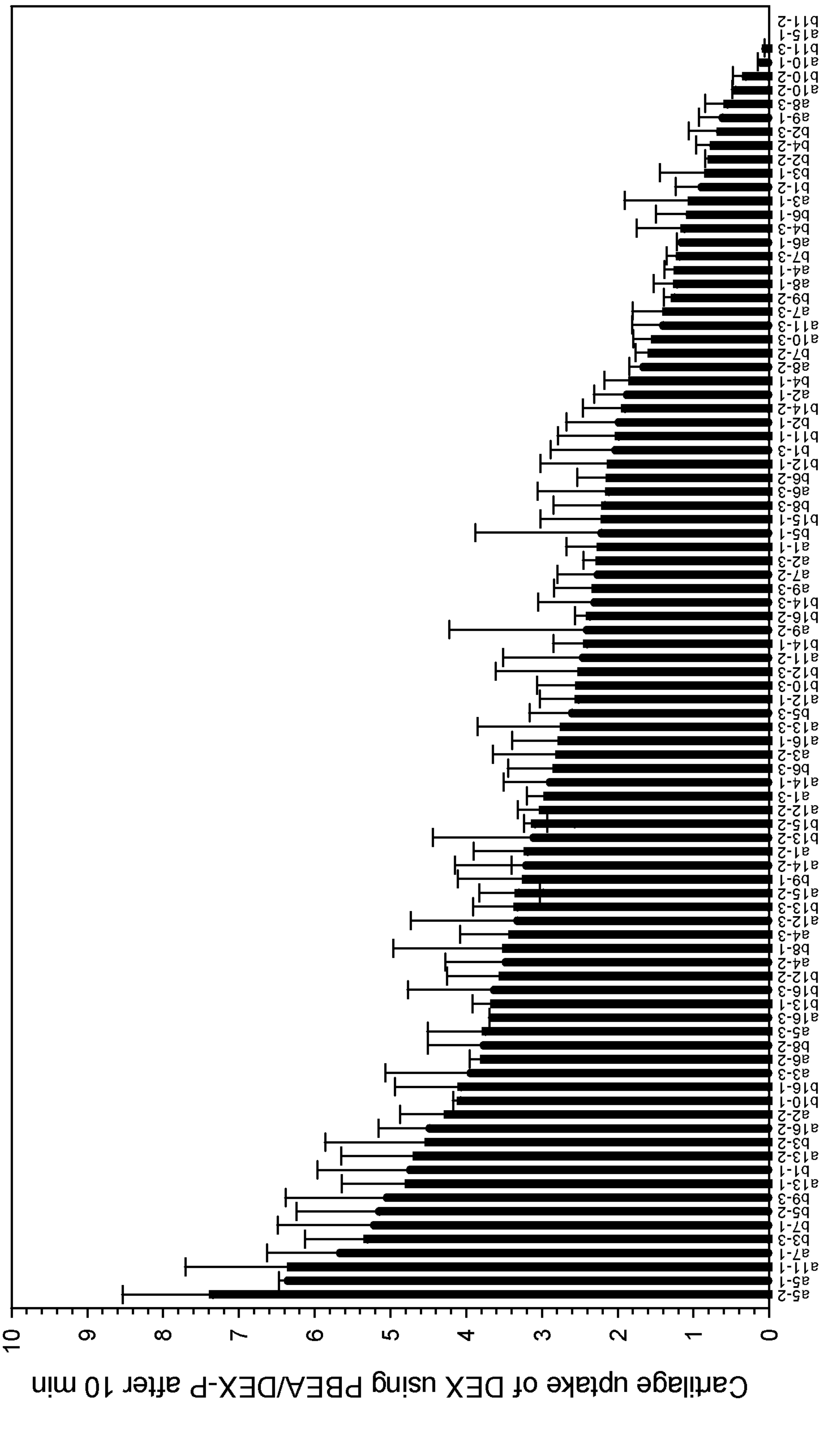
Cartilage uptake of DEX using PBEA/DEX-P after 10 min
0
1
2
3
4
5
6
7
8
9
10

Figure 7

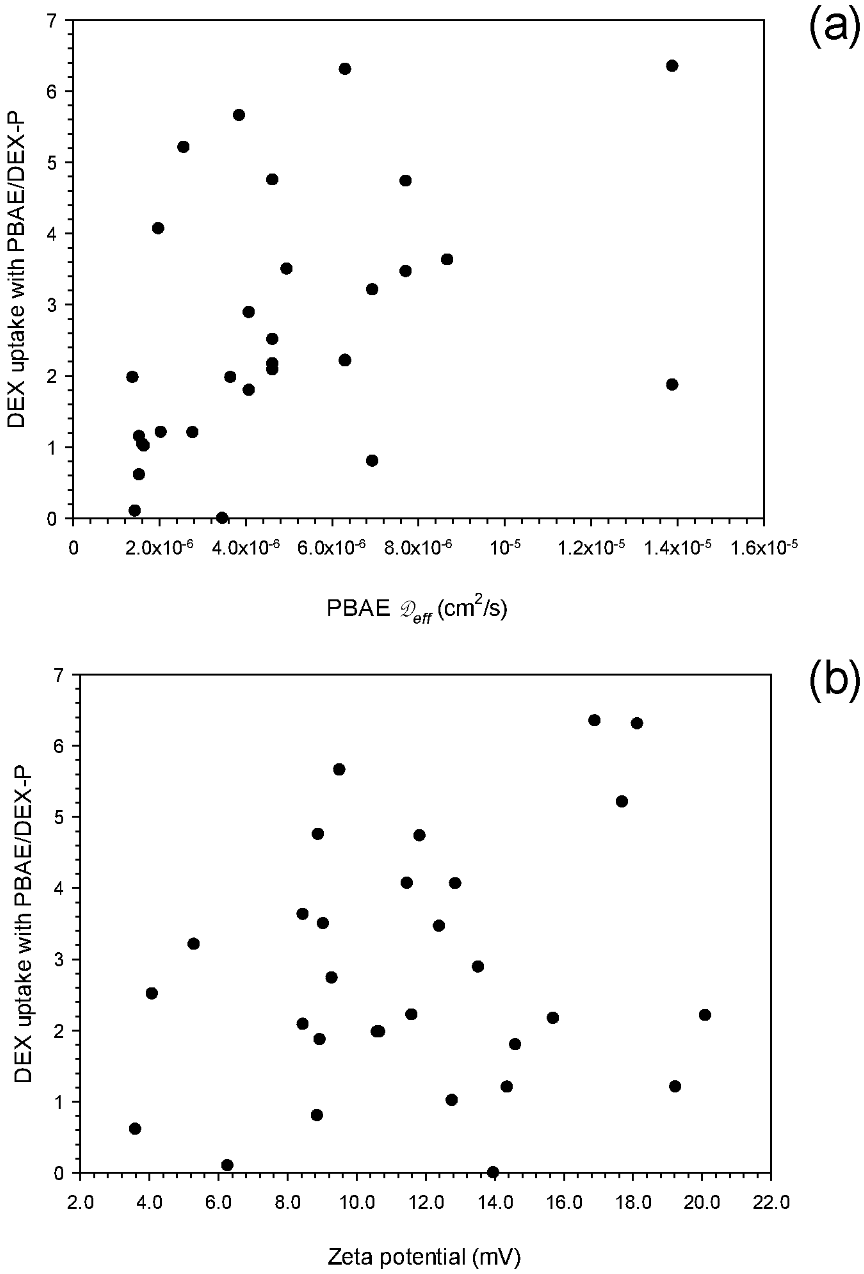
(a)
DEX uptake with PBAE/DEX-P
PBAE $\mathscr{D}_{eff}$ (cm$^2$/s)
0
2.0x10$^{-6}$
4.0x10$^{-6}$
6.0x10$^{-6}$
8.0x10$^{-6}$
10$^{-5}$
1.2x10$^{-5}$
1.4x10$^{-5}$
1.6x10$^{-5}$
(b)
DEX uptake with PBAE/DEX-P
Zeta potential (mV)
2.0
4.0
6.0
8.0
10.0
12.0
14.0
16.0
18.0
20.0
22.0

Figure 8

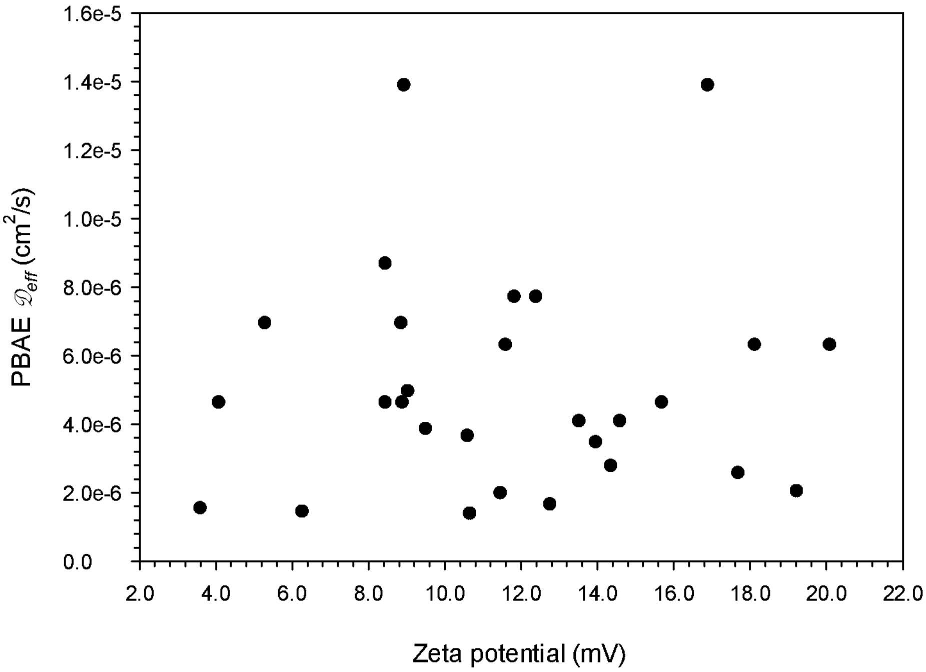


Figure 9

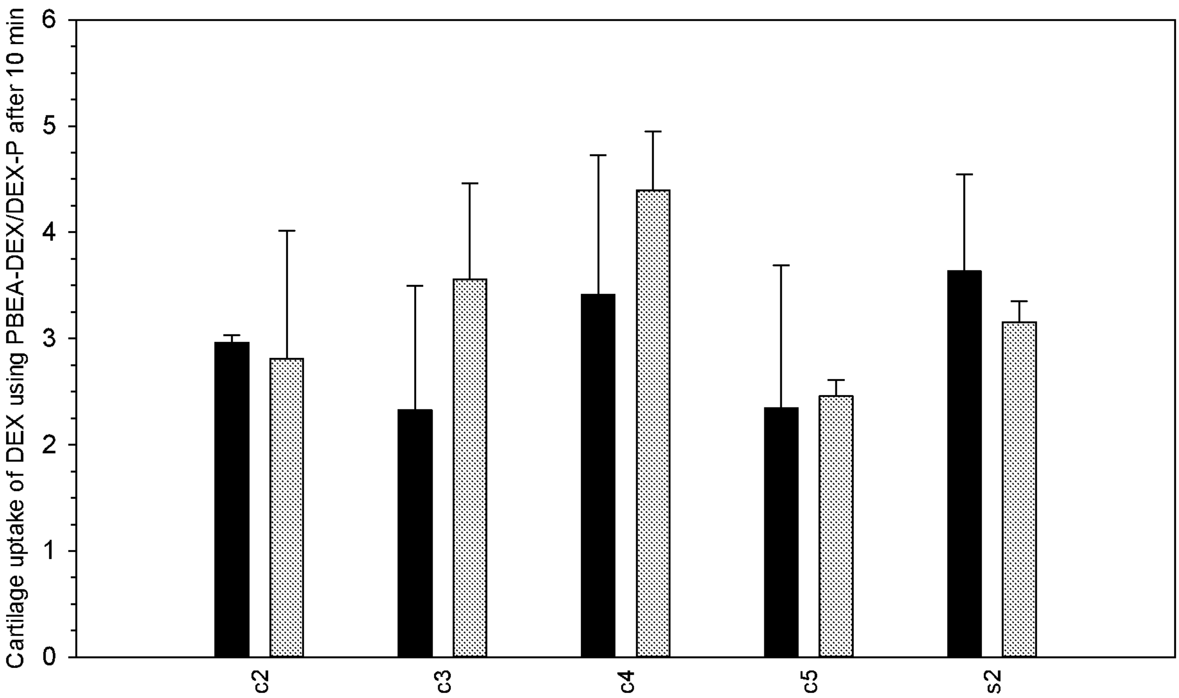

Figure 10

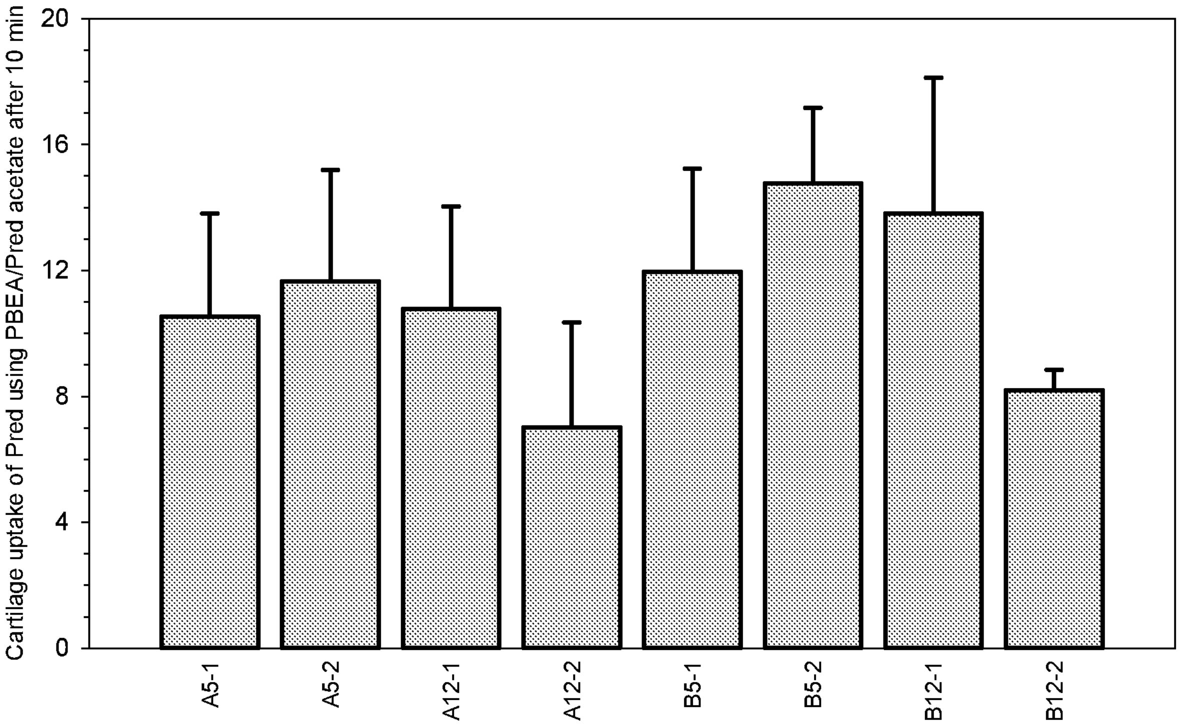
Cartilage uptake of Pred using PBEA/Pred acetate after 10 min
0
4
8
12
16
20
A5-1
A5-2
A12-1
A12-2
B5-1
B5-2
B12-1
B12-2

Figure 11

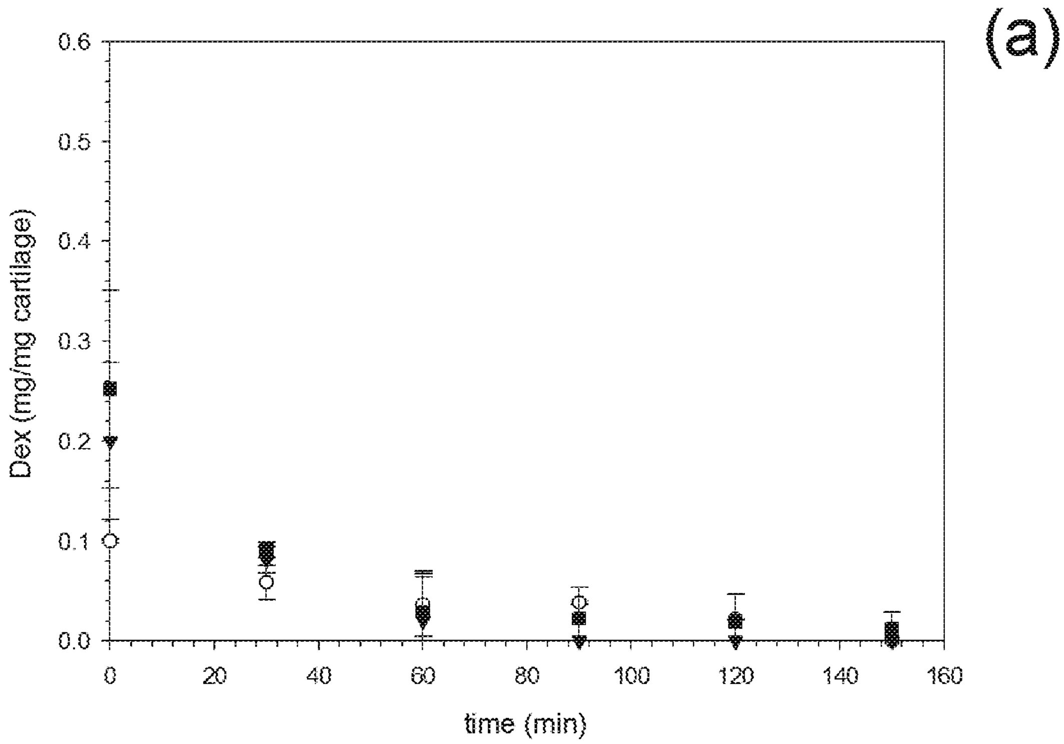
(a)
Dex (mg/mg cartilage)
0.0
0.1
0.2
0.3
0.4
0.5
0.6
0
20
40
60
80
100
120
140
160
time (min)

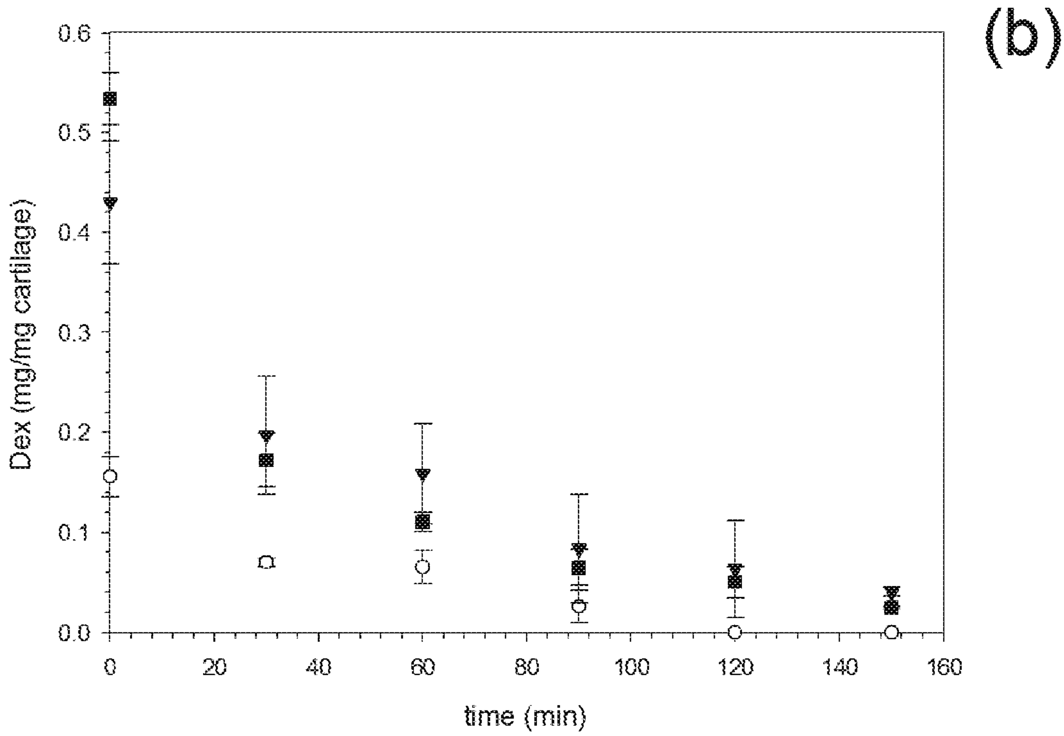
(b)
Dex (mg/mg cartilage)
0.0
0.1
0.2
0.3
0.4
0.5
0.6
0
20
40
60
80
100
120
140
160
time (min)

Figure 12

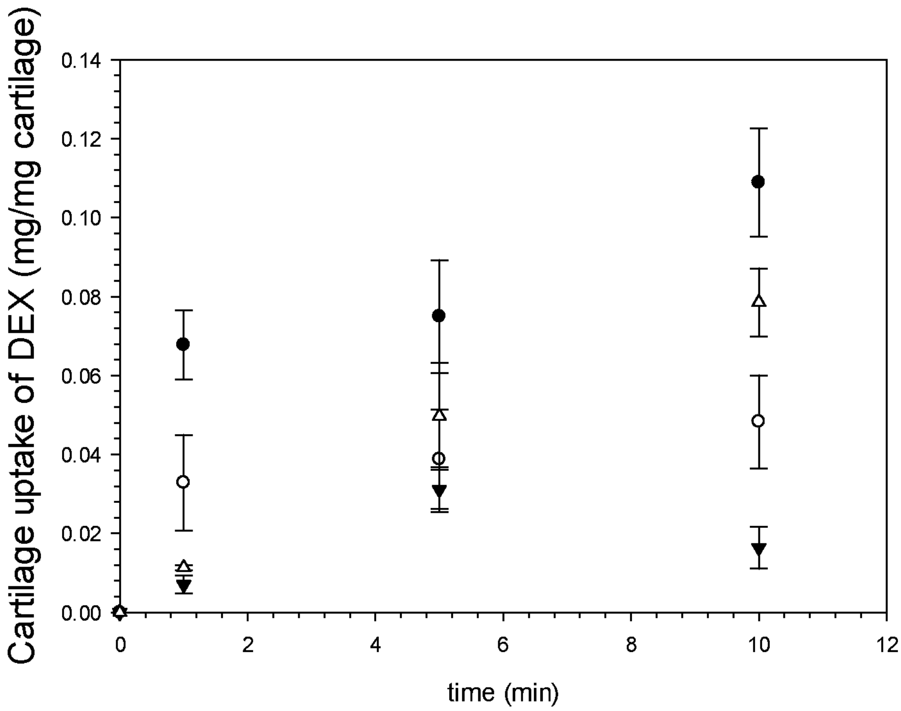


Figure 13

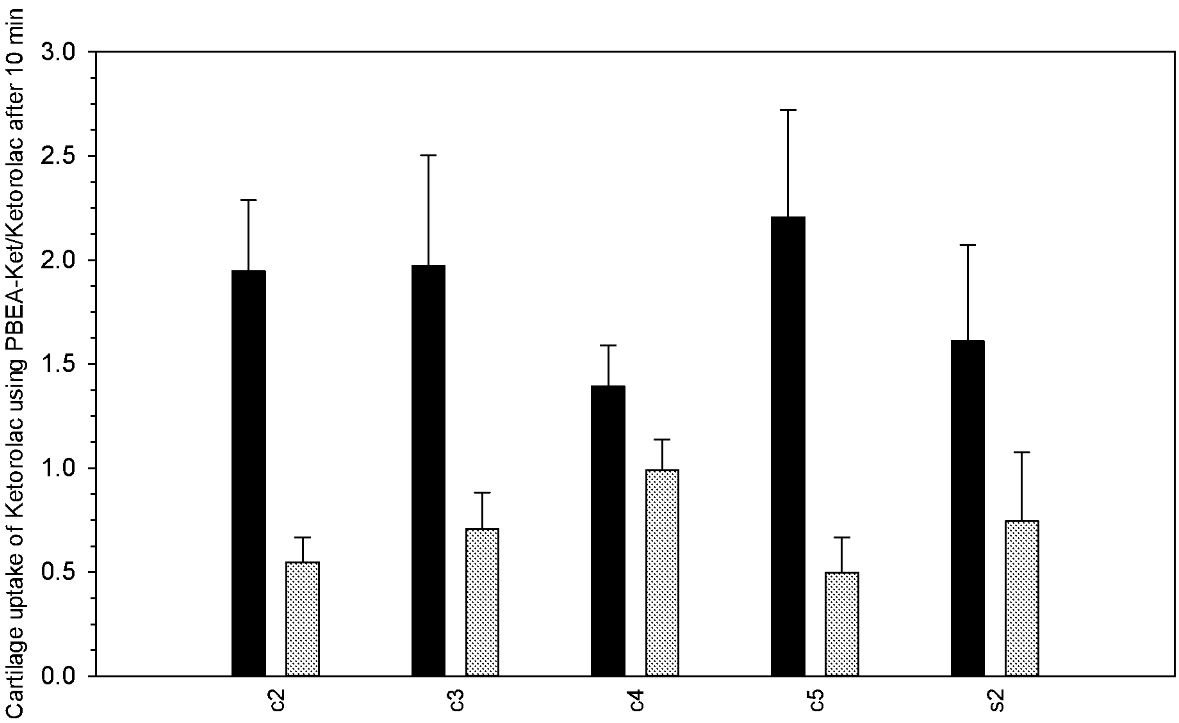

Figure 14

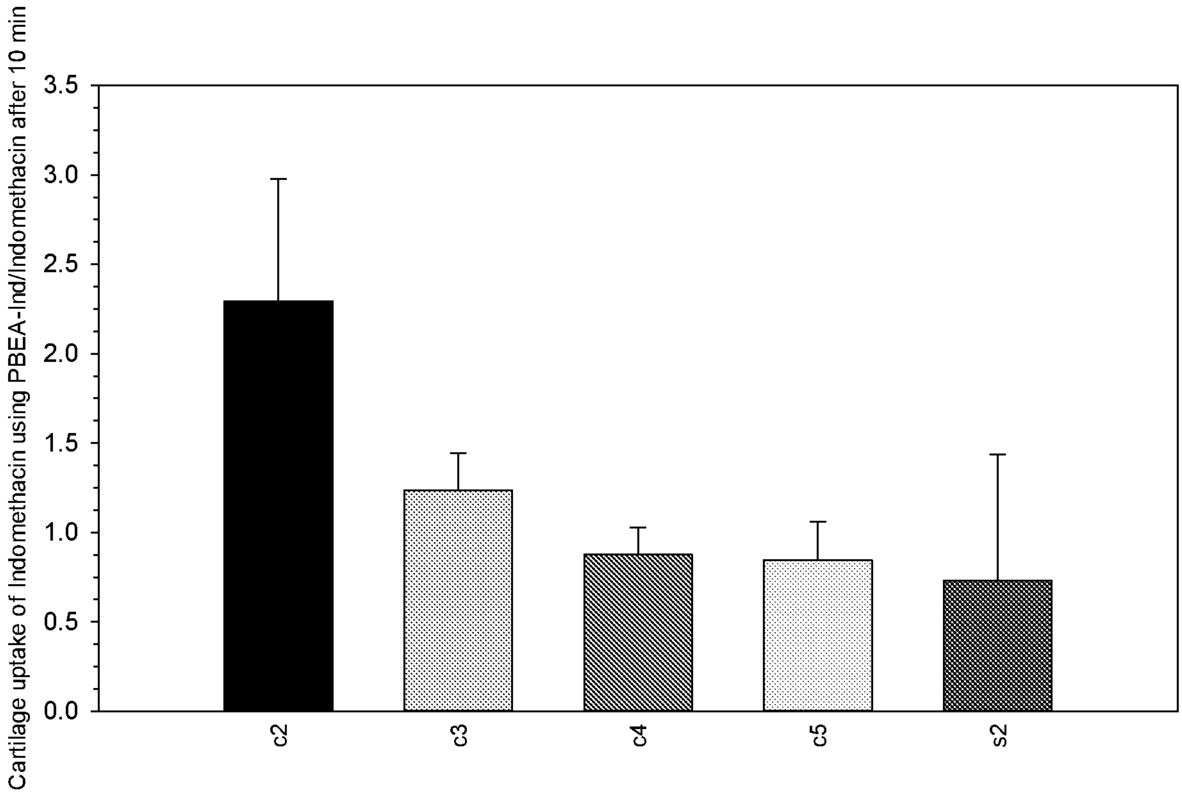


Figure 15

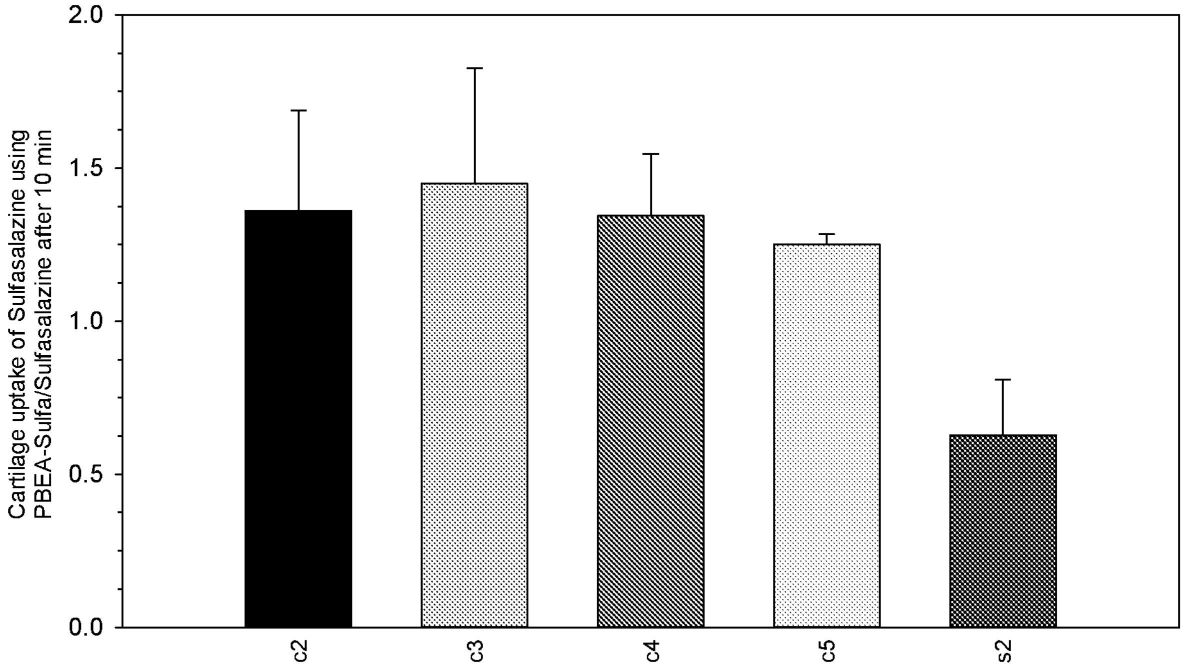

Figure 16

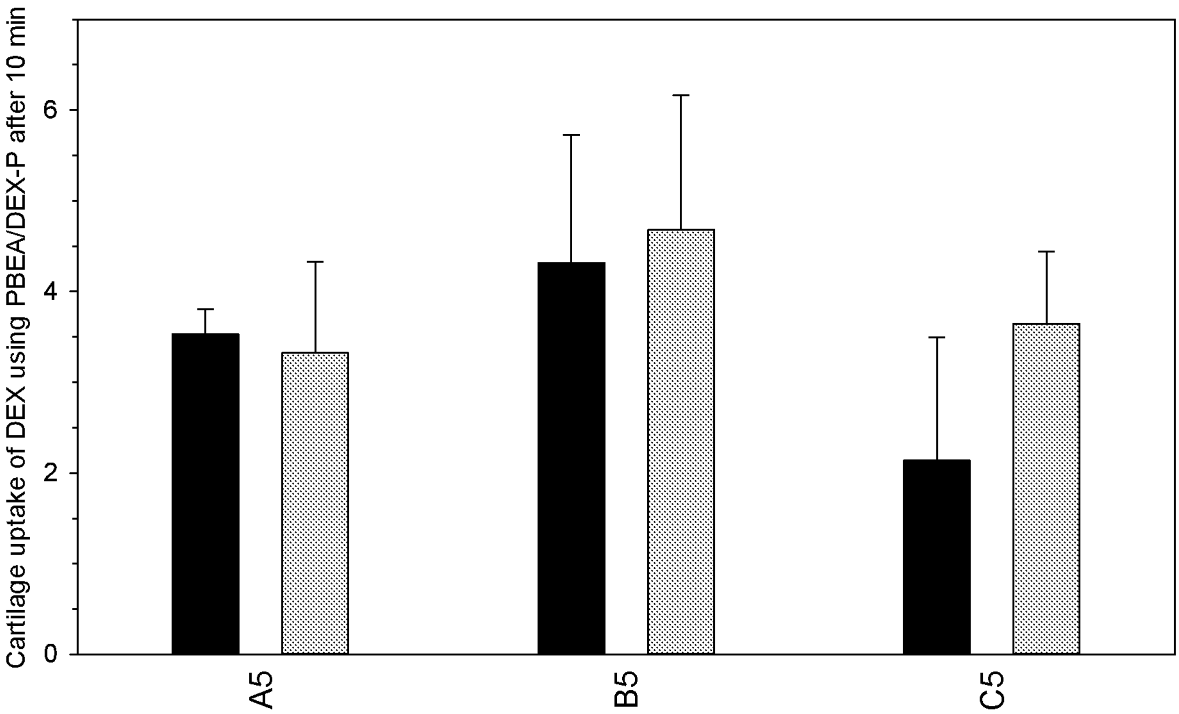


Figure 17

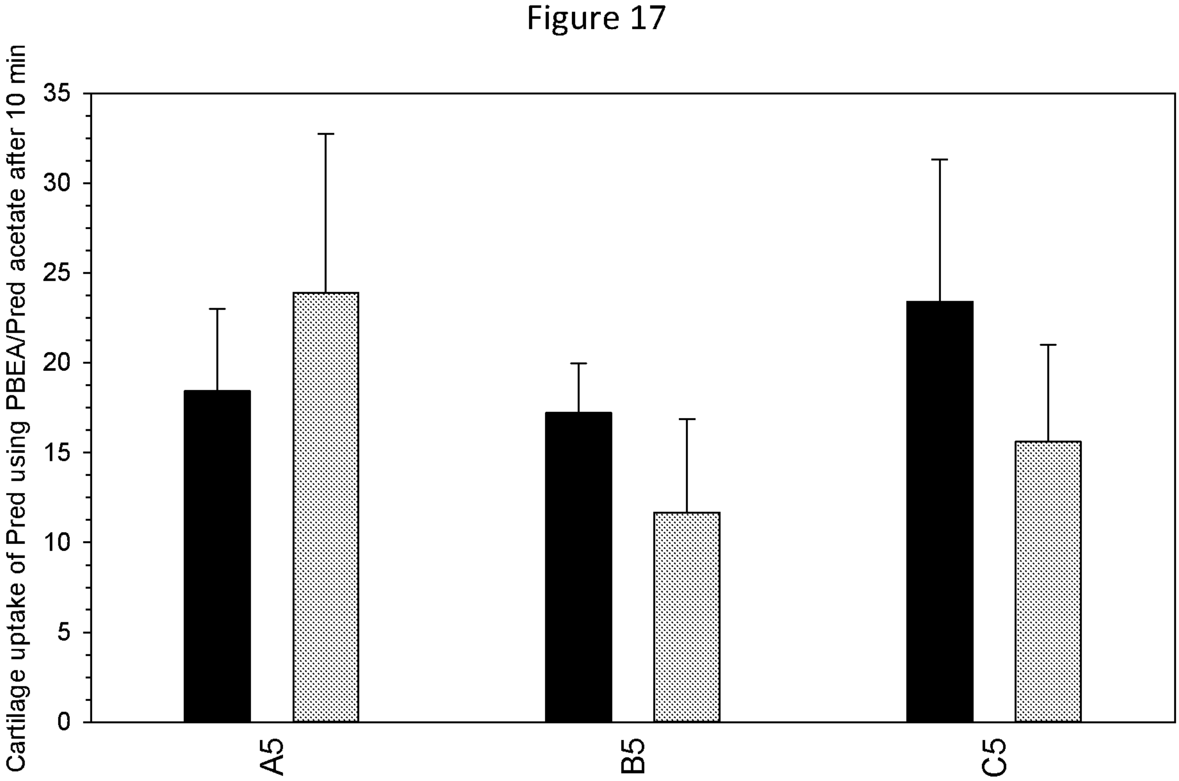

Figure 18

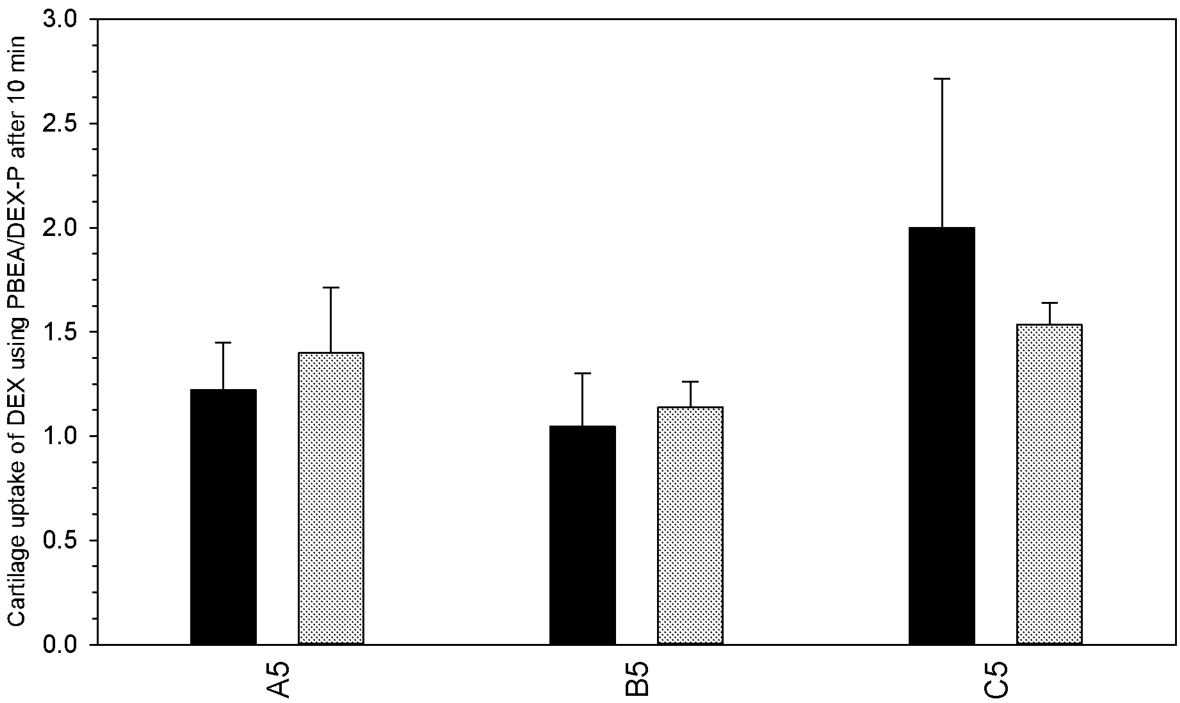


Figure 19

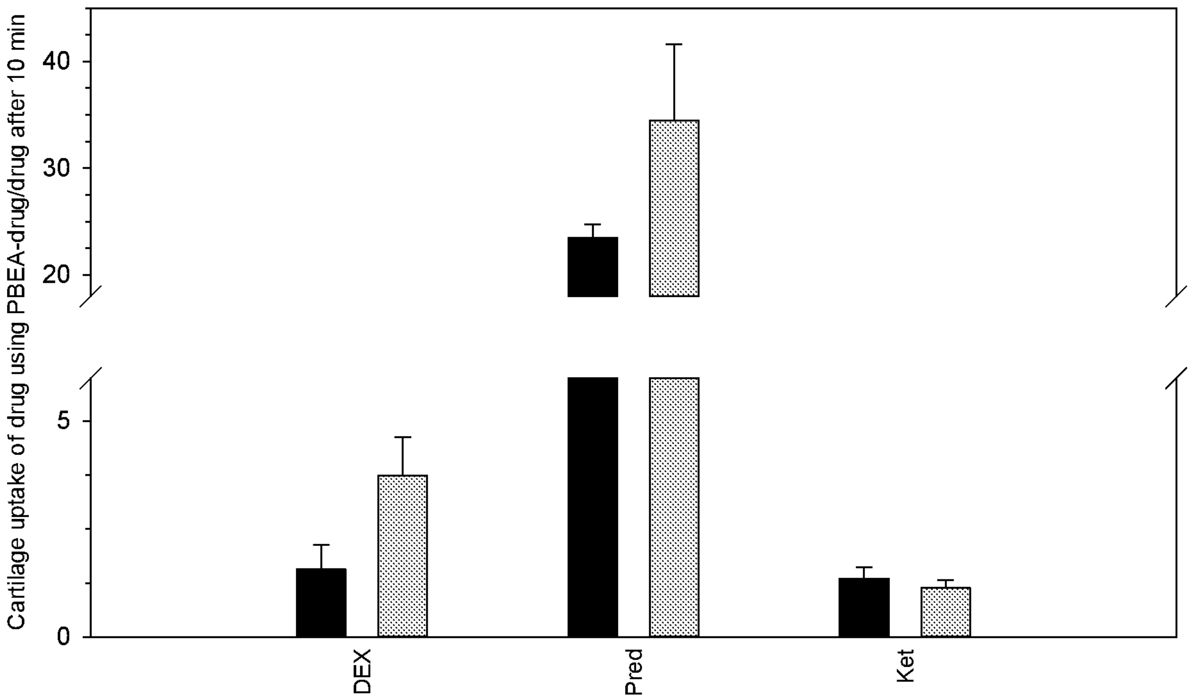

Figure 20

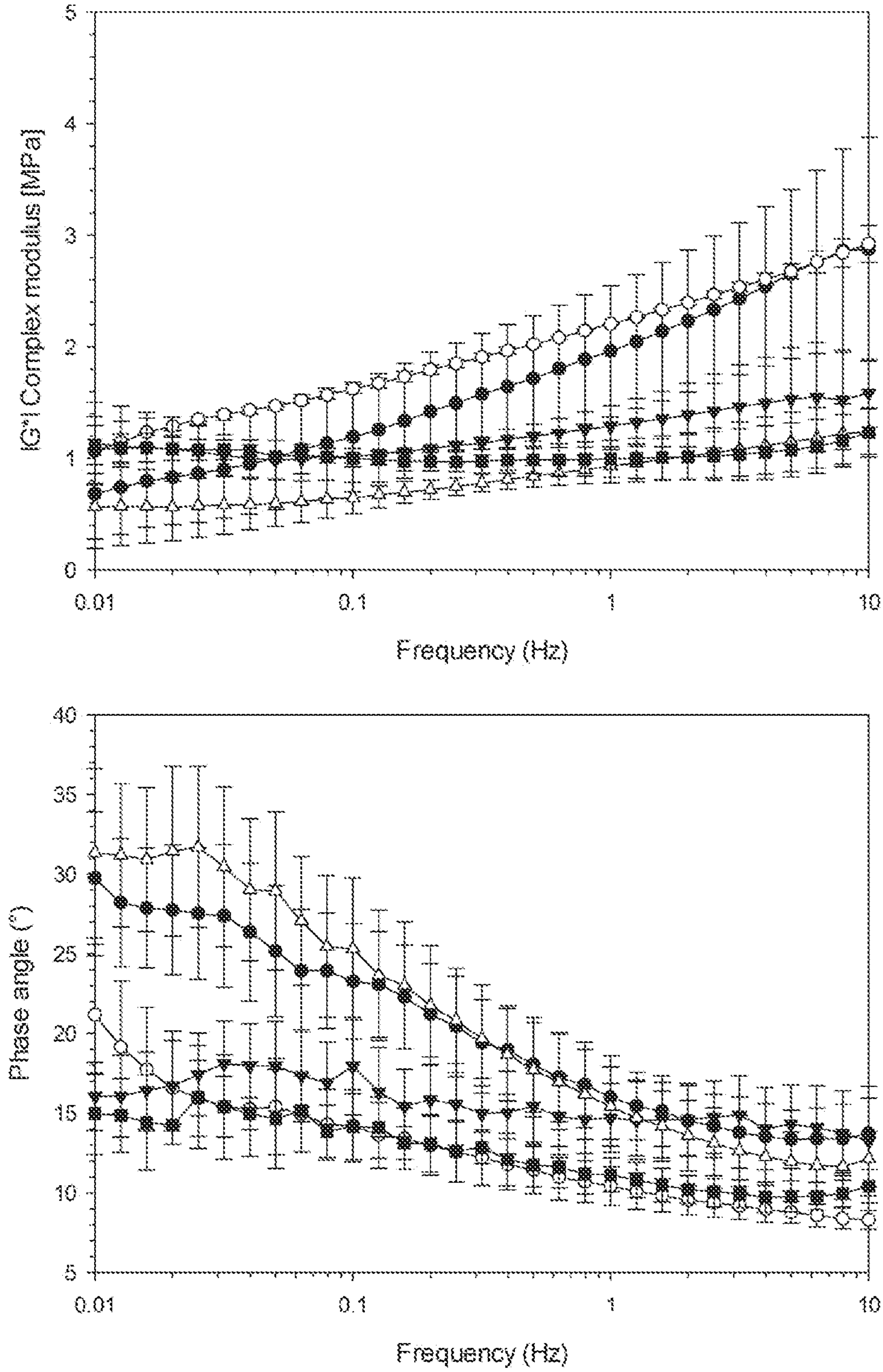
5
4
3
2
1
0
IG*I Complex modulus [MPa]
0.01
0.1
1
10
Frequency (Hz)
40
35
30
25
20
15
10
5
Phase angle (°)
0.01
0.1
1
10
Frequency (Hz)

Figure 21

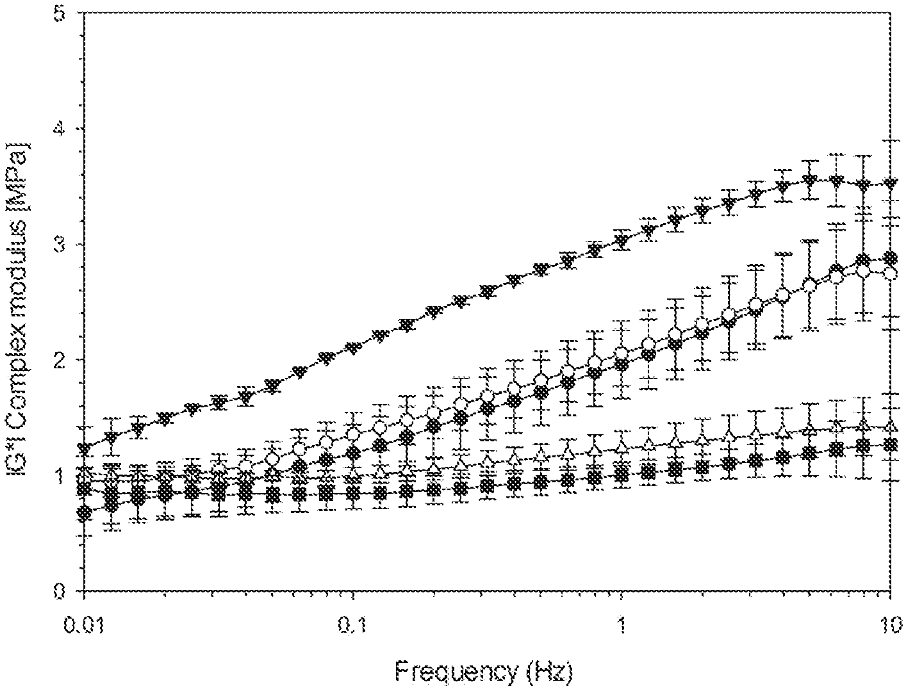
|G*| Complex modulus [MPa]
5
4
3
2
1
0
0.01
0.1
1
10
Frequency (Hz)

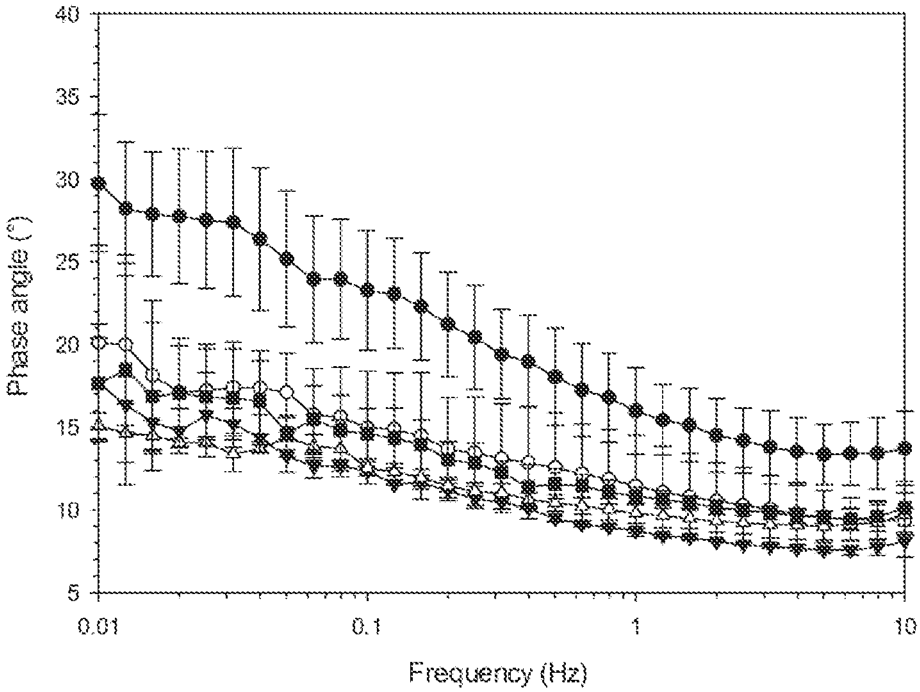
Phase angle (°)
40
35
30
25
20
15
10
5
0.01
0.1
1
10
Frequency (Hz)

Figure 22

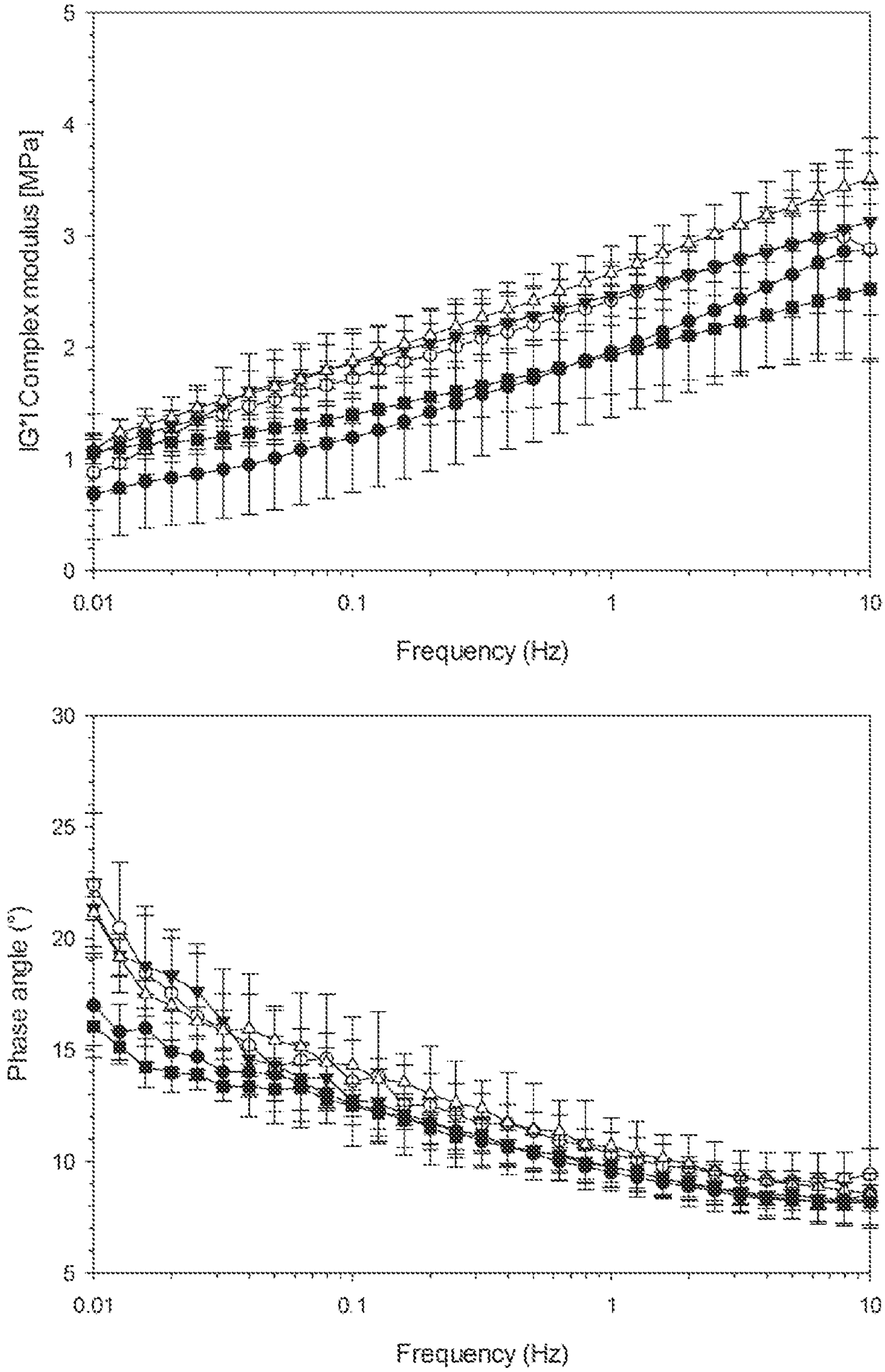
IG*I Complex modulus [MPa]
5
4
3
2
1
0
0.01
0.1
1
10
Frequency (Hz)
Phase angle (°)
30
25
20
15
10
5
0.01
0.1
1
10
Frequency (Hz)

Figure 23

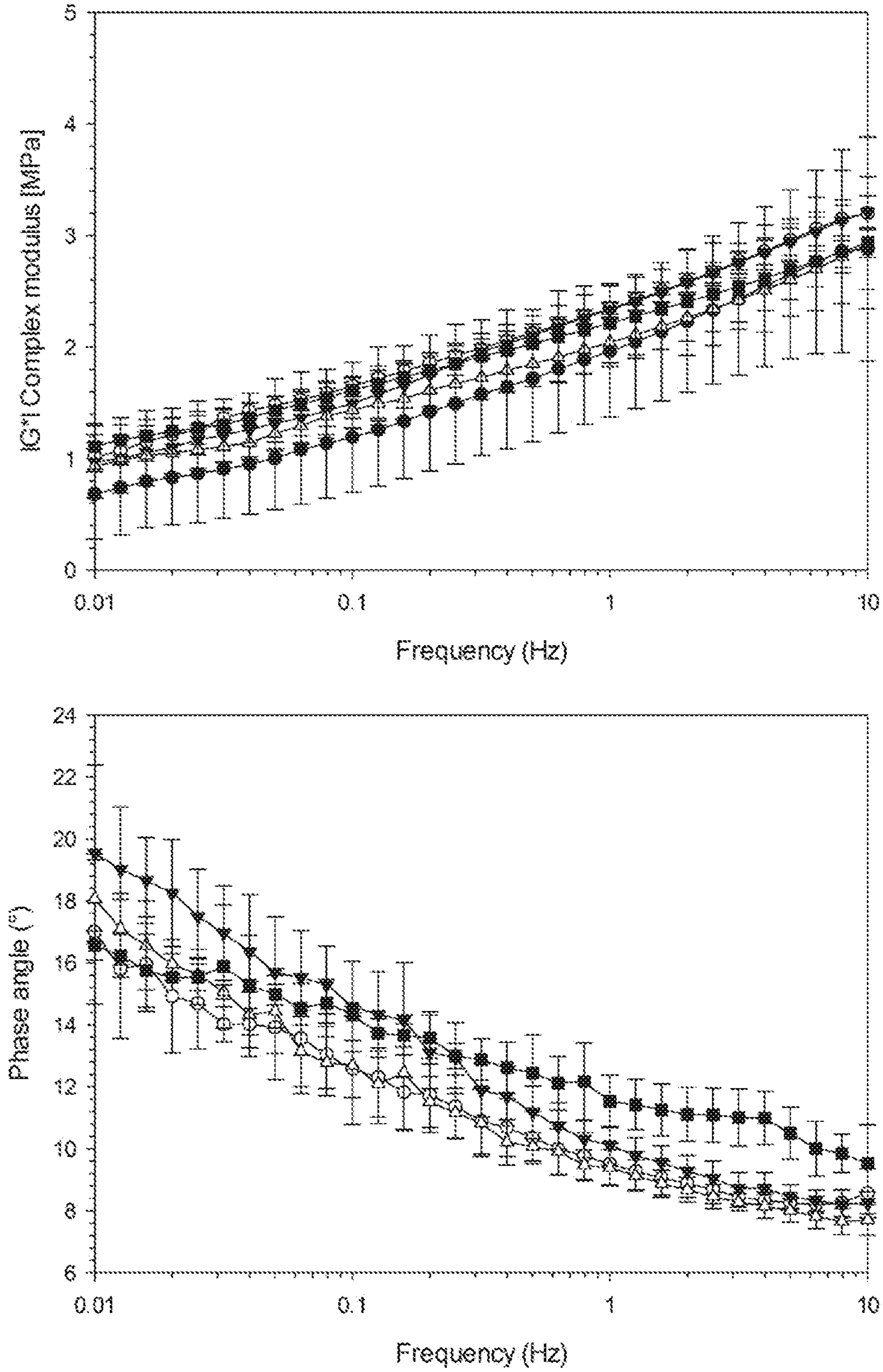
|G*| Complex modulus [MPa]
5
4
3
2
1
0
0.01
0.1
1
10
Frequency (Hz)
Phase angle (°)
24
22
20
18
16
14
12
10
8
6
0.01
0.1
1
10
Frequency (Hz)

Figure 24

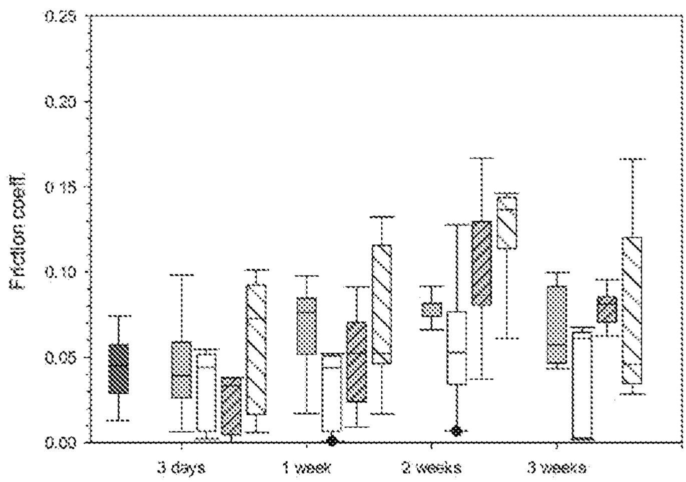


immature
with growth factors
without growth factors
growth factors conjugated to A5-e2
growth factors conjugated to A2-e1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 20140193508 A1


### Non-patent literature cited in the description


- **ANDERSON et al.** *Angew. Chem. Int. Ed.,* 2003, vol. 42, 3153-3158
- **KHAN et al.** *Arthritis and Rheumatism,* 2011, vol. 63 (11), 3417-3427
- **KHAN et al.** *Biomaterials,* 2013, vol. 34 (5), 1478-1487
- Remington's Pharmaceutical Sciences. Mack Publishing Co, 1985
- **FARNDALE et al.** *Biochim Biophys Acta,* 1986, vol. 883 (2), 173-177
- **CRANK J.** The mathematics of diffusion. Clarendon Press, 1975
- **NEU et al.** *Arthritis Rheum,* 2010, vol. 62 (9), 2680e7